# EUROPEAN PATENT SPECIFICATION

(11) **EP 3 331 897 B1**
(45) Date of publication and mention of the grant of the patent: **24.03.2021**
(21) Application number: 16751427.2
(22) Date of filing: 02.08.2016
(51) Int. Cl.: C07K 5/062, C07C 275/28, C07C 275/30, A61K 31/17, A61P 11/00, A61P 29/00

(54) **PHENYL UREA ANALOGS AS FORMYL PEPTIDE RECEPTOR 1 (FPR1) SELECTIVE AGONISTS**
PHENYLHARNSTOFFANALOGA ALS SELEKTIVE AGONISTEN DES FORMYLPEPTIDREZEPTORS 1 (FPR1)
ANALOGUES DE PHÉNYLURÉE EN TANT QU'AGONISTES SÉLECTIFS DE RÉCEPTEUR DE PEPTIDE FORMYLE (FPR1)

(30) Priority: 05.08.2015 US 201562201387 P
(43) Date of publication of application: 13.06.2018
(73) Proprietor: ALLERGAN, INC., Irvine, CA 92612 (US)
(72) Inventor: DUONG, Tien T., Rancho Santa Margarita, California 92688 (US); BEARD, Richard L., Newport Beach, California 92660 (US); GARST, Michael E., Newport Beach, California 92660 (US)
(74) Representative: Hoffmann Eitle
(86) International application number: PCT/US2016/045114
(87) International publication number: WO 2017/023907

(56) References cited:
- WO-A1-2013/062947

## Description

### FIELD OF THE INVENTION

The present invention relates to phenyl urea derivatives, pharmaceutical compositions containing them and these compounds and compositions for use as pharmaceuticals as modulators of N-formyl peptide receptor(s) (FPR(s)), such as modulators of the N-formyl peptide receptor 1 (FPR1) and/or the N-formyl peptide receptor 2 (FPR2; also known as FPRL-1 or ALXA4), or as selective modulators of FPR1 relative to FPR2. The invention relates specifically to these compounds and their pharmaceutical compositions for use in treating disorders associated with FPR modulation, such as FPR1 and/or FPR2 agonism, or selective agonism of FPR1 relative to FPR2.

### BACKGROUND OF THE INVENTION

The FPR family belongs to the seven transmembrane domain G-protein-coupled receptor (GPCR) family. There are three members of this family in humans, including FPR1 and FPR2. FPRs are critical regulators of host defense in phagocytosis, and are considered highly relevant factors for the chemotaxis of immune cells. In view of their ability to promote the resolution of inflammation, these receptors represent an important "pro-resolutionary" molecular target for the development of new therapeutic agents in diseases or conditions involving excessive inflammatory responses.

WO 2014/138037 A1 discloses methods of treating ocular inflammatory diseases by administering a pharmaceutical composition comprising an FPR agonist; WO 2014/138046 A1 discloses methods of treating dermal inflammation and dermal diseases by administering a pharmaceutical composition comprising an FPR agonist; and US 2013/0109866 discloses compounds of the general structure below (with the variable "R" groups as defined therein) as FPR modulators for the treatment of a variety of diseases or conditions, including ocular and dermal inflammatory diseases and conditions: 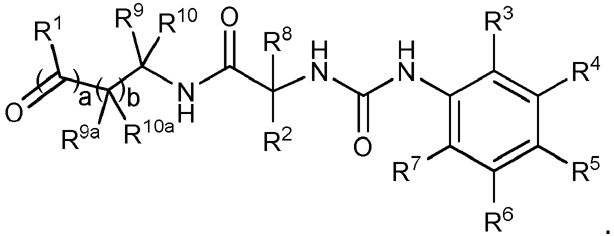 FPR2 is expressed predominantly on inflammatory cells, such as monocytes and neutrophils, as well as on T cells, and has been shown to play a critical role in leukocyte trafficking during inflammation and human pathology (see Chiang N, Serhan CN, Dahlen, S, Drazen JM, Hay DWP, Rovati E, Shimizu T, Yokomizo T, Brink, C. The lipoxin receptor ALX: Potent ligand-specific and stereoselective actions in vivo. Pharmacological Reviews 2006; 58: 463-519).

FPRs are also expressed by immune cells of the central nervous system (CNS), and FPR expression is up-regulated during bacterial meningitis. Lack of FPR1 and FPR2 leads to more severe inflammation and higher mortality in mice infected with *Streptococcus pneumonia* within the CNS, suggesting that these FPRs play an important role in the innate response against this pathogen in the CNS (Oldekamp, S. et al., Immunology, 143(3), pp. 447-461, 2014).

FPR1 and FPR2 mediate rapid neutrophil mobilization to accelerate wound healing, as shown in Listeria-infected mice. These FPRs sense pathogen-derived chemotactic ligands and recognize host-derived chemotactic peptides in inflammation and injury. The FPRs promote the healing of sterile skin wounds in mice by initiating neutrophil infiltration (Liu, M. et al., PLoS One, 9(6): e90613, 2014). FPRs have also been shown to guide the first wave of neutrophil infiltration in livers of Listeria-infected mice to effectively eliminate the invading pathogen (Liu, M. et al., Sci. Rep., Vol 2, pp. 786, 2012). The FPRs appear to play a prominent role in regulating the hepatic inflammatory response after LPS induced liver injury; for example, FPR1 and FPR2 deficiency has been associated with increased inflammation and enhanced liver injury after LPS stimulation (Giebeler, A. et al., PLoS One, 9(6): e100522, 2014).

During intestinal mucosal injury, a complex array of proinflammatory and protective mechanisms regulates inflammation and severity. Controlling inflammatory responses and promoting epithelial restitution and barrier recovery requires secretion of anti-inflammatory mediators (Babbin, B.A. et al., J. Immunol., 208, 181(7), pp. 5035-5044). FPR1, a chemo-attractant receptor expressed mainly on leukocytes, is expressed in epithelia, and an FPR1/NADPH oxidase (NOX1)-dependent redox signaling pathway that promotes mucosal wound repair has been delineated in intestinal epithelia. Specific gut microbiota stimulate FPR1 on intestinal epithelial cells, generating reactive oxygen species via enterocyte NOX1, causing rapid phosphorylation of focal adhesion kinase (FAK) and extracellular signal-regulated kinase mitogen-activated protein kinase, which together stimulate migration and proliferation of enterocytes adjacent to colonic wounds. FPR1 was thus identified as a pattern recognition receptor for perceiving the enteric microbiota that promote mucosal wound repair by generating reactive oxygen species from the enterocyte NOX1. (See Leoni, G. et al., J. Clin. Invest., Vol 123, pp. 443-454, 2013; Alam, A. et al., Mucosal Immunol., 2014, 7(3), pp. 645-655). Regarding FPR2, the role of the ALX/FPR2 receptor-ligand interaction in regulating dextran sulfate sodium (DDS)-induced colitis revealed that treatment with an ALX/FPR2 agonist, 15-epi-lipoxin A4, reverses the enhanced sensitivity of annexin A1 (-/-) mice to DDS-colitis (Babbin, B.A. et al., supra).

FPR1 is also functionally expressed on human lens epithelial cells and appears to have a direct functional role in lens development and maintenance (Schneider et al., J. Biol. Chem., V287, pp. 40779-40792, 2012).

We have discovered phenyl urea derivatives that exhibit selectivity for FPR1 relative to FPR2. To our knowledge, the present invention provides the first compounds to selectively modulate FPR1.

Other phenyl urea derivatives are known. For example:
Journal of Combinatorial Chemistry (2007), 9(3), 370-385 teaches a thymidinyl dipeptide urea library with structural similarity to the nucleoside peptide class of antibiotics:

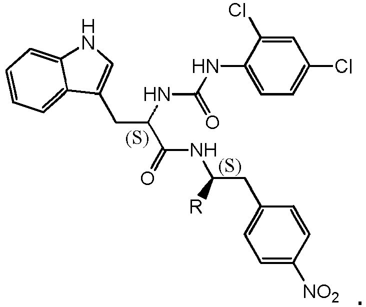

Helvetica Chimica Acta (1998), 81(7), 1254-1263 teaches the synthesis and spectroscopic characterization of 4-chlorophenyl isocyanate (1-chloro-4-isocyanatobenzene) adducts with amino acids as potential dosimeters for the biomonitoring of isocyanate exposure:

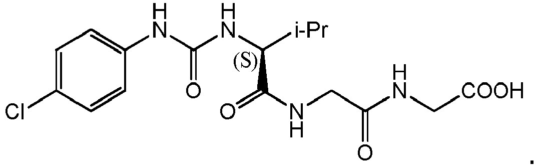

Yingyong Huaxue (1990), 7(1), 1-9 teaches the structure-activity relationships of di- and tripeptide sweeteners and of L-phenyl alanine derivatives:

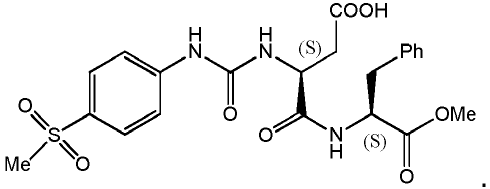

FR 2533210 discloses L-phenyl alanine derivatives as synthetic sweeteners:

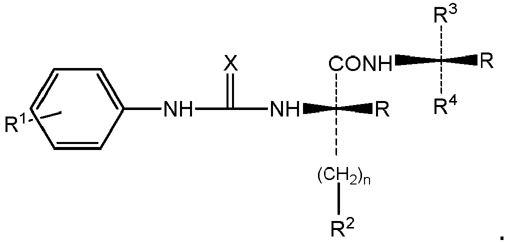

The following compounds are known as registered with Chemical Abstract Services (CAS), identified herein by structure and CAS registry number:

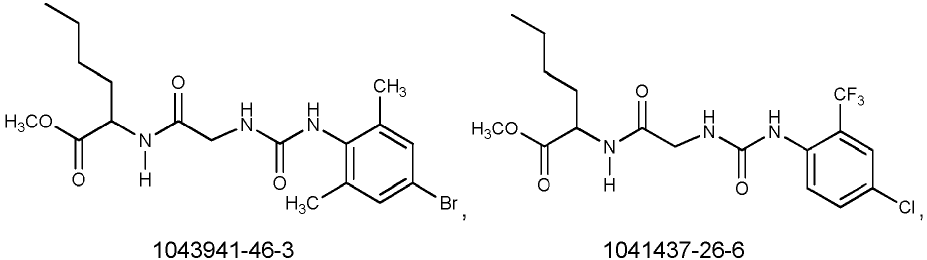

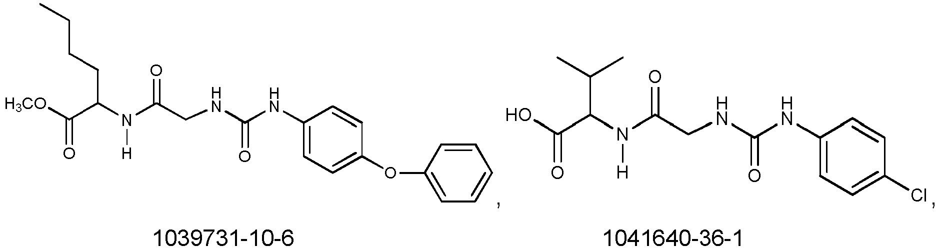

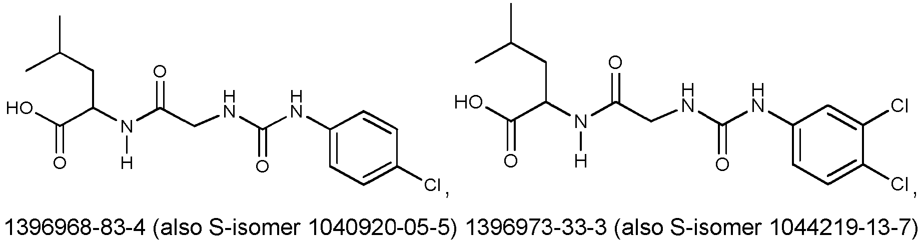

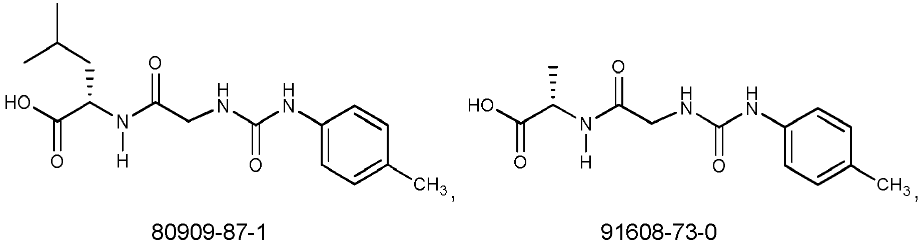

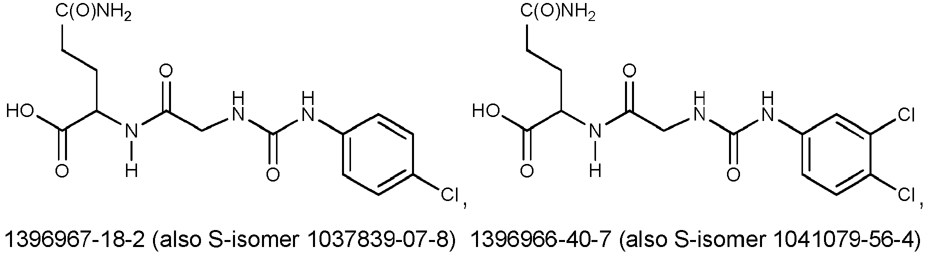

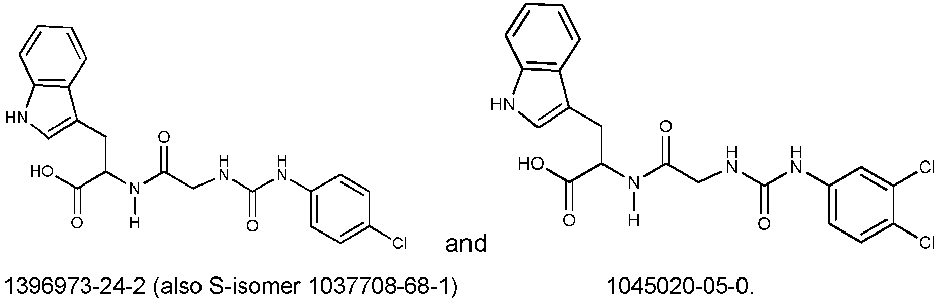

### SUMMARY OF THE INVENTION

A group of phenyl urea derivatives, which are potent and selective FPR1 modulators, has been discovered. As such, the compounds described herein are useful in treating a wide variety of disorders associated with inflammatory conditions modulated, at least in part, by the FPR receptor. The disorders may be associated with the modulation of FPR1 and/or FPR2, or with selective modulation of FPR1 relative to FPR2. The term "modulator" as used herein includes, but is not limited to: receptor agonist, antagonist, inverse agonist, inverse antagonist, partial agonist, and partial antagonist.

This invention describes compounds of Formula I and la, which modulate FPR biological activity. The compounds in accordance with the present invention are thus of use in medicine, for example, in the treatment of mammalian subjects, including humans, with diseases and/or conditions that are alleviated by FPR modulation (such as FPR1 and/or FPR2 agonism, or FPR1 agonism, or selective agonism of FPR1 relative to FPR2).

In one aspect, the invention provides a compound represented by **Formula I:** 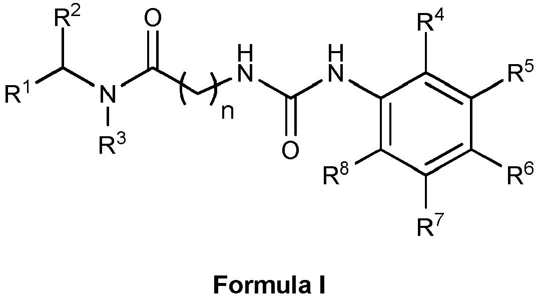 wherein:
R¹ is -COOH or -C(O)OR^{a}, R^{a} is optionally substituted C₁₋₆ alkyl, wherein said optional alkyl substituent is selected from OH, halogen, -OC₁₋₈alkyl and -(O(CH₂)₁₋₈)_{q}-OC₁₋₈ alkyl; and q is 1, 2, 3, 4, 5 or 6;
R² is optionally substituted C₁₋₆ alkyl, wherein said optional alkyl substituent is selected from -OH, -SH, -OC₁₋₆ alkyl, -SC₁₋₆ alkyl, -COOH, -C(O)OC₁₋₆ alkyl, -C(O)NH₂, C₃₋₈ cycloalkyl, C₃₋₈ cycloalkenyl, C₆₋₁₀ aryl, and heterocycle;
R³ is H or unsubstituted C₁₋₆ alkyl;
R⁴ is H, C₁₋₆ alkyl, C₁₋₆ haloalkyl, C₃₋₈ cycloalkyl, C₃₋₈ cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
R⁵ is H, C₁₋₆ alkyl, C₁₋₆ haloalkyl, halogen, -S(O)ₘR⁹ or -C(O)R¹⁰;
R⁶ is C₁₋₆ alkyl, C₁₋₆ haloalkyl, C₃₋₈ cycloalkyl, C₃₋₈ cycloalkenyl, C₆₋₁₀ aryl, heterocycle, halogen, -S(O)ₘR⁹, -C(O)R¹⁰ or -OR¹¹;
R⁷ is H, C₁₋₆ alkyl, C₁₋₆ haloalkyl, halogen, -S(O)ₘR⁹ or -C(O)R¹⁰;
R⁸ is H, C₁₋₆ alkyl, C₁₋₆ haloalkyl, C₃₋₈ cycloalkyl, C₃₋₈ cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
each R⁹ is independently -OH, C₁₋₆ alkyl or C₆₋₁₀ aryl;
each R¹⁰ is independently -OH, C₁₋₆ alkyl or C₆₋₁₀ aryl;
each R¹¹ is independently H, C₁₋₈ alkyl, C₃₋₈ cycloalkyl, C₃₋₈ cycloalkenyl, C₆₋₁₀ aryl or heterocycle;
each R¹² is independently H, C₁₋₈ alkyl, C₃₋₈ cycloalkyl, C₃₋₈ cycloalkenyl, C₆₋₁₀ aryl or heterocycle;
each R¹³ is independently H or C₁₋₈ alkyl;
each m is independently 1 or 2; and
n is 1, 2 or 3;
or a single enantiomer thereof;
or a mixture of enantiomers thereof;
or a tautomer of the foregoing;
or pharmaceutically acceptable salt of the foregoing.
provided that the compound is not:

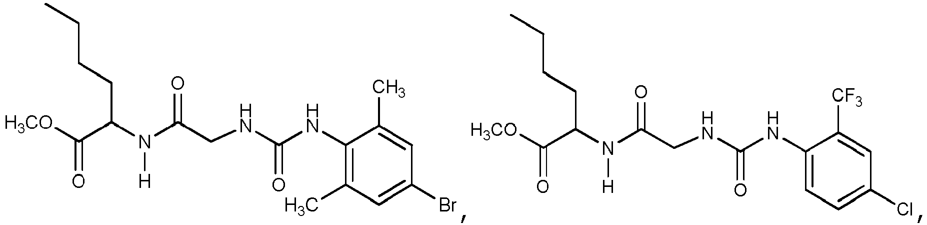

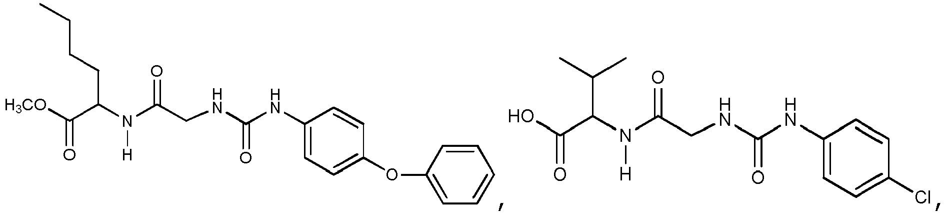

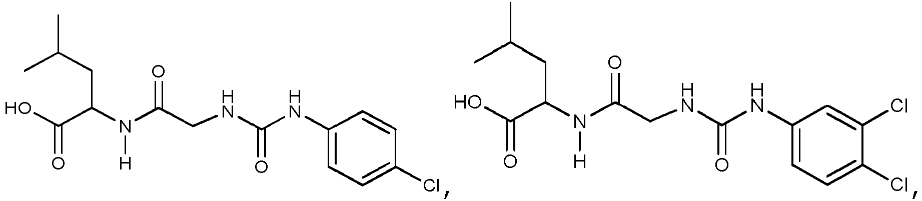

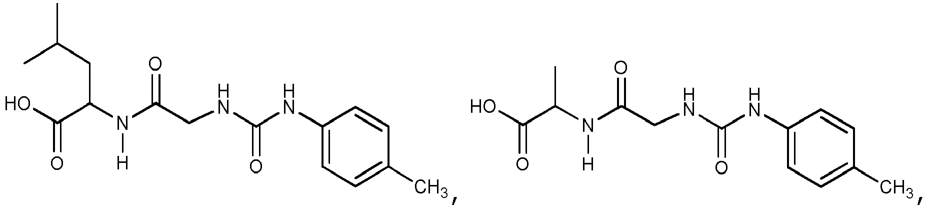

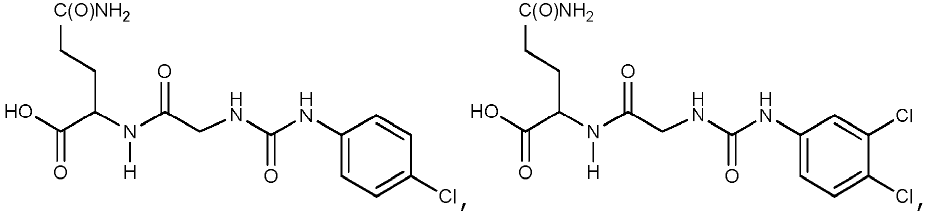

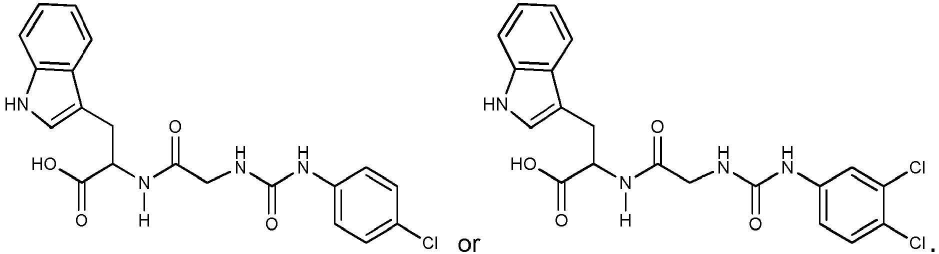

In another aspect of the invention, there are provided pharmaceutical compositions comprising a therapeutically effective amount of at least one compound of the invention described herein in a pharmaceutically acceptable carrier.

In another aspect of the invention, there are provided compounds that selectively agonize FPR1 compared to FPR2. In further aspects, the compound shows at least 10-fold selectivity for FPR1 compared to FPR2, or at least 20-fold selectivity for FPR1 compared to FPR2. In yet further aspects, the compound shows at least 100-fold selectivity, at least 200-fold selectivity, or at least 300-fold selectivity for FPR1 compared to FPR2. In the preceding aspects, the selectivity is reported based on the ratio of the EC₅₀ for agonizing FPR2 to the EC₅₀ for agonizing FPR1.

In yet another aspect of the invention, there are provided compounds or compositions for use in methods for treating disorders associated with FPR modulation, such as FPR1 and/or FPR2 agonism, or selective agonism of FPR1 relative to FPR2. Such methods can be performed, for example, by administering to a subject in need thereof a pharmaceutical composition containing a therapeutically effective amount of at least one compound of the invention. In some aspects, the disorder is an inflammatory disease or condition. In further aspects, the inflammatory disease or condition is an ocular inflammatory disease or condition, such as dry eye or post-surgical inflammation, including post-cataract surgical inflammation. In yet further aspects, the inflammatory disease or condition is a dermal inflammatory disease or condition, such as psoriasis or rosacea. In further aspects, the method involves treating dermal wounds and promotes the healing of dermal wounds. In further aspects, the inflammatory disease or condition is a systemic inflammatory disease or condition. In yet further aspects, the disease or condition is an autoimmune disease or condition. In further aspects, the subject is a mammal, such as a human or non-human primate.

### DETAILED DESCRIPTION OF THE INVENTION

It is to be understood that both the foregoing general description and the following detailed description are exemplary and explanatory only and are not restrictive of the invention claimed. As used herein, the use of the singular includes the plural unless specifically stated otherwise.

The term "alkyl", as used herein, refers to saturated, monovalent or divalent hydrocarbon moieties having linear or branched moieties or combinations thereof. Alkyl groups typically contain 1 to 6 carbon atoms (i.e., C₁₋₆ alkyl), but may contain a variable number of carbon atoms as specified. For example, an alkyl group may comprise 1 to 4 carbon atoms (i.e., C₁₋₄ alkyl), or 1 to 3 carbon atoms (i.e., C₁₋₃ alkyl). Unsubstituted C₁₋₄ alkyl includes methyl, ethyl, n-propyl, isopropyl, n-butyl, isobutyl, sec-butyl and t-butyl. Unsubstituted C₁₋₃ alkyl includes methyl, ethyl, n-propyl and isopropyl.

The term "alkylene" as used herein refers to a bivalent saturated aliphatic radical derived from an alkene by opening of the double bond, or from an alkane by removal of two hydrogen atoms from one or from different carbon atoms. An alkylene may comprise 1 to 8 carbon atoms (i.e., C₁₋₈ alkylene), for example, a C₁ alkylene is methylene (-CH₂-); a C₂ alkylene is ethylene (-CH₂CH₂-), and so on.

The term "cycloalkyl", as used herein, refers to a monovalent or divalent group of 3 to 8 carbon atoms (i.e., C₃₋₈ cycloalkyl) derived from a saturated cyclic hydrocarbon. Cycloalkyl groups can be monocyclic or polycyclic.

The term "cycloalkenyl", as used herein, refers to a monovalent or divalent group of 3 to 8 carbon atoms (i.e., C₃₋₈ cycloalkenyl) derived from a saturated cycloalkyl having one or more double bonds. Cycloalkenyl groups can be monocyclic or polycyclic.

The term "heterocycle" as used herein, refers to a 3 to 10 membered ring, which can be aromatic (i.e., a heteroaryl) or non-aromatic, saturated or unsaturated, containing at least one heteroatom selected from O, N and S, or combinations of at least two thereof, interrupting the carbocyclic ring structure. The heterocyclic ring can be interrupted by one or more C=O; the S and/or N heteroatom can be oxidized. Heterocycles can be monocyclic or polycyclic.

The term "aryl" as used herein, refers to an aromatic hydrocarbon ring containing 6 to 10 carbon atoms (i.e., C₆₋₁₀ aryl). Aryl can be monocyclic or polycyclic.

The term "halogen", as used herein, refers to an atom of fluorine, chlorine, bromine, and/or iodine.

The term "amine" or "amino" as used herein, represents a group of formula "-NR^{x}R^{y}", wherein R^{x} and R^{y} can be the same or independently H, alkyl, aryl, cycloalkyl, cycloalkenyl or heterocyclyl, as defined above.

The term "amide" as used herein, represents a group of formula "-C(O)N(R^{x})(R^{y})" or "-NR^{x}C(O)R^{y}" wherein R^{x} and R^{y} can be the same or independently H, alkyl, aryl, cycloalkyl, cycloalkenyl or heterocyclyl, as defined above.

The term "sulfonamide" as used herein, represents a group of formula "-S(O)₂N(R^{x})(R^{y})" or "-NR^{x}S(O)₂R^{y} wherein R^{x} and R^{y} can be the same or independently H, alkyl, aryl, cycloalkyl, cycloalkenyl or heterocyclyl, as defined above.

The term "aldehyde" as used herein, represents a group of formula "-C(O)H".

The term "ester" as used herein, represents a group of formula "-C(O)O(R^{x})", wherein R^{x} is alkyl, aryl, cycloalkyl, cycloalkenyl or heterocyclyl, as defined above.

The term "thioester" as used herein, represents a group of formula "-C(O)S(R^{x})", wherein R^{x} is alkyl, aryl, cycloalkyl, cycloalkenyl or heterocyclyl, as defined above.

The term "ketone" as used herein, represents a group of formula "-C(O)R^{x}" wherein R^{X} is alkyl, aryl, cycloalkyl, cycloalkenyl or heterocyclyl, as defined above.

The term "hydroxyl" as used herein, represents a group of formula "-OH".

The term "thiol" as used herein, represents a group of formula "-SH".

The term "carbonyl" as used herein, represents a group of formula "-C(O)-".

The term "carboxyl" as used herein, represents a group of formula "-C(O)O-".

The term "carboxylic acid" as used herein, represents a group of formula "-C(O)OH".

The term "carboxylate" as used herein, represents a group of formula "-C(O)O".

The term "sulfoxide" as used herein, represents a group of formula "-S(O)-".

The term "sulfonyl" as used herein, represents a group of formula "-SO₂-".

The term "sulfate" as used herein, represents a group of formula "-OS(O)₂O⁻".

The term "nitro" as used herein, represents a group of formula "-NO₂".

The term "nitrile" as used herein, represents a group of formula "-CN".

The term "ether" as used herein, represents a group of formula "-OR^{x}", wherein R^{x} is alkyl, aryl, cycloalkyl, cycloalkenyl or heterocyclyl, as defined above.

The term "thioether" as used herein, represents a group of formula "-SR^{x}", wherein R^{x} is alkyl, aryl, cycloalkyl, cycloalkenyl or heterocyclyl, as defined above.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "pharmaceutically acceptable salts" refers to salts or complexes that retain the desired biological activity of compounds of the invention, and exhibit minimal or no undesired toxicological effects. The "pharmaceutically acceptable salts" according to the invention include therapeutically active, non-toxic base or acid salt forms, which the compounds of Formula I and la are able to form.

The term "carboxylate isostere", as used herein, refers to a group that replaces a carboxylic acid, such as a group selected from sulfonate, sulfonic acid, phosphonate, phosphonic acid, phosphoric acid, boronic acid and unsubstituted or substituted heterocycle, wherein said heterocycle is selected from tetrazole, imidazole, thiazole, oxazole, isoxazole, oxadiazole, thiadiazole, triazole, thiophene, pyrazole and pyrole; and wherein said heterocycle substituent is selected from unsubstituted and substituted C₁₋₈ alkyl, wherein said alkyl substituent is selected from OH and halogen.

The term "therapeutically effective amount" means the amount of a pharmaceutical composition that will elicit a biological or medical response in a subject in need thereof that is being sought by the researcher, veterinarian, medical doctor or other clinician.

In one embodiment, the present invention provides a compound represented by **Formula I:** 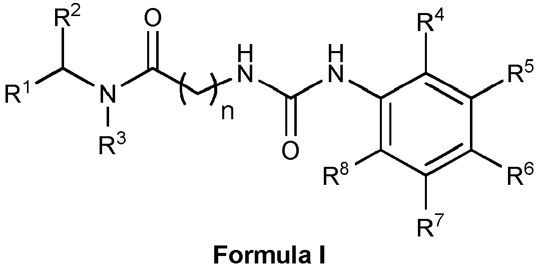 wherein:
R¹ is -COOH or -C(O)OR^{a}, R^{a} is optionally substituted C₁₋₆ alkyl, wherein said alkyl optional substituent is selected from OH, halogen, -OC₁₋₈ alkyl and -(O(CH₂)₁₋₈)_{q}-OC₁₋₈ alkyl; and q is 1, 2, 3, 4, 5 or 6;
R² is optionally substituted C₁₋₆ alkyl, wherein said optional alkyl substituent is selected from -OH, -SH, -OC₁₋₆ alkyl, -SC₁₋₆ alkyl, -COOH, -C(O)OC₁₋₆ alkyl, -C(O)NH₂, C₃₋₈ cycloalkyl, C₃₋₈ cycloalkenyl, C₆₋₁₀ aryl, and heterocycle;
R³ is H or unsubstituted C₁₋₆ alkyl;
R⁴ is H, C₁₋₆ alkyl, C₁₋₆ haloalkyl, C₃₋₈ cycloalkyl, C₃₋₈ cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
R⁵ is H, C₁₋₆ alkyl, C₁₋₆ haloalkyl, halogen, -S(O)ₘR⁹ or -C(O)R¹⁰;
R⁶ is C₁₋₆ alkyl, C₁₋₆ haloalkyl, C₃₋₈ cycloalkyl, C₃₋₈ cycloalkenyl, C₆₋₁₀ aryl, heterocycle, halogen, -S(O)ₘR⁹, -C(O)R¹⁰ or -OR¹¹;
R⁷ is H, C₁₋₆ alkyl, C₁₋₆ haloalkyl, halogen, -S(O)ₘR⁹ or -C(O)R¹⁰;
R⁸ is H, C₁₋₆ alkyl, C₁₋₆ haloalkyl, C₃₋₈ cycloalkyl, C₃₋₈cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
each R⁹ is independently -OH, C₁₋₆ alkyl or C₆₋₁₀ aryl;
each R¹⁰ is independently -OH, C₁₋₆ alkyl or C₆₋₁₀ aryl;
each R¹¹ is independently H, C₁₋₈ alkyl, C₃₋₈ cycloalkyl, C₃₋₈ cycloalkenyl, C₆₋₁₀ aryl or heterocycle;
each R¹² is independently H, C₁₋₈ alkyl, C₃₋₈ cycloalkyl, C₃₋₈ cycloalkenyl, C₆₋₁₀ aryl or heterocycle;
each R¹³ is independently H or C₁₋₈ alkyl;
each m is independently 1 or 2; and
n is 1, 2 or 3;
or a single enantiomer thereof;
or a mixture of enantiomers thereof;
or a tautomer of the foregoing;
or pharmaceutically acceptable salt of the foregoing.
provided that the compound is not:

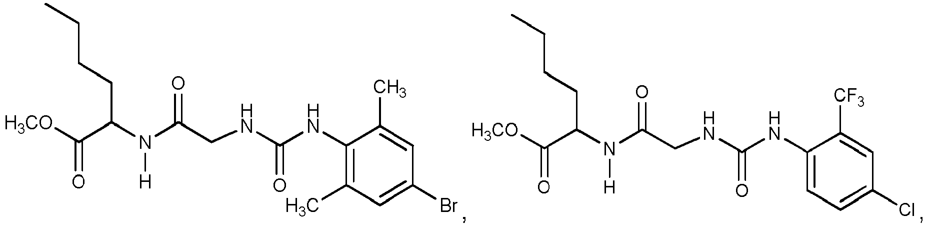

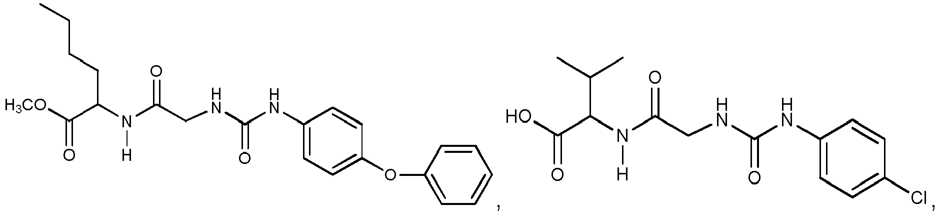

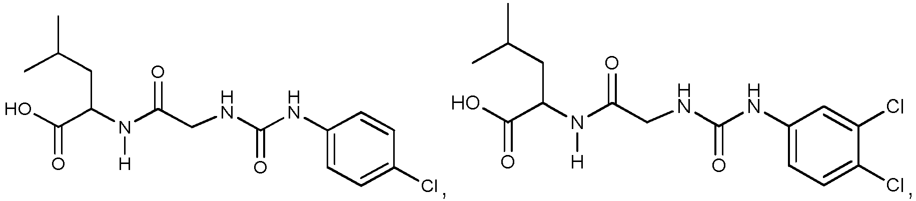

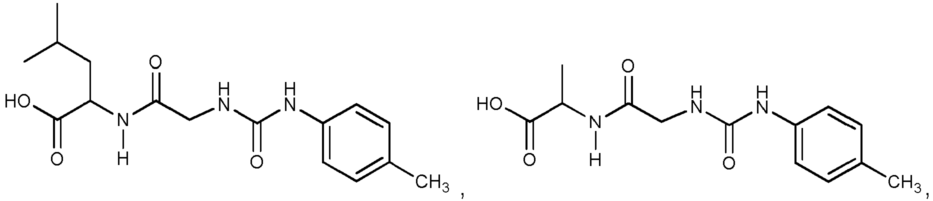

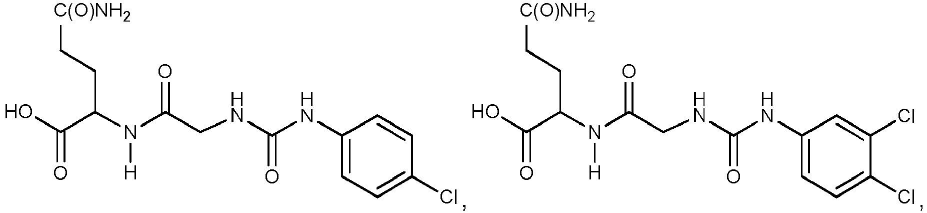

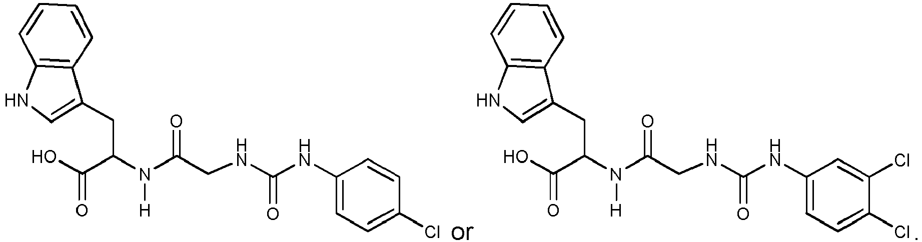

In some embodiments, there are provided compounds of Formula I, wherein R¹ is -COOH or -C(O)OR^{a}, wherein R^{a} is unsubstituted or substituted C₁₋₆ alkyl; wherein said alkyl substituent is selected from OH, halogen, -OC₁₋₈ alkyl and -(O(CH₂)₁₋₈)_{q}-OC₁₋₈ alkyl; and q is 1, 2, 3, 4, 5 or 6. In further embodiments, there are provided compound of Formula I, wherein R¹ is -COOH or -C(O)OR^{a}, and R^{a} is unsubstituted C₁₋₆ alkyl. In other embodiments, there are provided compounds of Formula I, wherein R¹ is -COOH.

In some embodiments, there are provided compounds of Formula I wherein R² is a sidechain derived from a naturally occurring amino acid, such as glycine, alanine, valine, leucine, isoleucine, serine, cysteine, threonine, methionine, asparagine, glutamine, aspartic acid, glutamic acid, lysine, arginine, phenylalanine, tyrosine, tryptophan or histidine. In some embodiments, R² is -CH₃, -CH(CH₃)₂, -CH₂CH(CH₃)₂, -CH(CH₃)CH₂CH₃, -CH₂OH, -CH(OH)CH₃,-CH₂SH, -CH₂CH₂SCH₃, -CH₂C(O)NH₂, -CH₂CH₂C(O)NH₂, -CH₂C(O)OH, -CH₂CH₂C(O)OH, -CH₂CH₂CH₂CH₂NH₂, -CH₂CH₂CH₂NHC(NH)NH₂, 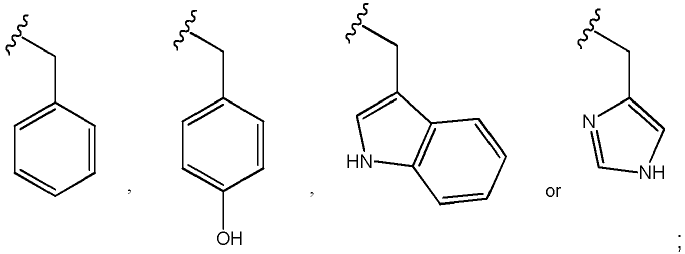 or a salt thereof.

In some embodiments, there are provided compounds of Formula I, wherein R² is unsubstituted C₁₋₆ alkyl or benzyl. In some embodiments, R² is unsubstituted C₁₋₆ alkyl. In other embodiments, R² is unsubstituted C₁₋₄ alkyl, such as methyl, ethyl, n-propyl, isopropyl, n-butyl, tert-butyl, isobutyl or sec-butyl. In another embodiment, R² is unsubstituted C₁₋₃ alkyl, such as methyl, ethyl, n-propyl or isopropyl. In other embodiments, R² is unsubstituted benzyl.

In some embodiments, there are provided compounds of Formula I, wherein R³ is H or unsubstituted C₁₋₃ alkyl. In some embodiments, R³ is H. In another embodiment, R³ is unsubstituted C₁₋₃ alkyl, such as methyl, ethyl, n-propyl or isopropyl. In yet another embodiment, R³ is methyl.

In some embodiments, there are provided compounds of Formula I, wherein n is 1 or 2. In another embodiment, n is 1. In yet other embodiment, n is 2.

In some embodiments, there are provided compounds of Formula I, wherein R⁴ is H, F or C₁₋₆ haloalkyl; R⁵ is H, F or C₁₋₆ haloalkyl; R⁷ is H, F or C₁₋₆ haloalkyl; and R⁸ is H, F or C₁₋₆ haloalkyl. In another embodiment, there are provided compounds wherein R⁴ is H, F or C₁₋₆ fluoroalkyl; R⁵ is H, F or C₁₋₆ fluoroalkyl; R⁷ is H, F or C₁₋₆ fluoroalkyl; and R⁸ is H, F or C₁₋₆ fluoroalkyl. In a further embodiment, there are provided compounds wherein R⁴ is H, F or C₁₋₆ perfluoroalkyl; R⁵ is H, F or C1-6 perfluoroalkyl; R⁷ is H, F or C₁₋₆ perfluoroalkyl; and R⁸ is H, F or C₁₋₆ perfluoroalkyl. In yet a further embodiment, there are provided compounds of Formula I wherein R⁴ is H, F or CF₃; R⁵ is H, F or CF₃; R⁷ is H, F or CF₃; and R⁸ is H, F or CF₃. In another embodiment, R⁴ is H or F; R⁵ is H or F; R⁷ is H or F; and R⁸ is H or F. In a further embodiment, there are provided compounds of Formula I, wherein at least one of R⁴ and R⁸ is H. In other embodiment, there are provided compounds of Formula I, wherein each of R⁴, R⁵, R⁷ and R⁸ is H.

In some embodiments, there are provided compounds of Formula I, wherein R⁶ is C₁₋₆ haloalkyl or halogen. In one embodiment, R⁶ is C₁₋₆ haloalkyl or Br. In another embodiment, R⁶ is C₁₋₆ fluoroalkyl or bromine. In a further embodiment, R⁶ is C₁₋₆ perfluoroalkyl or bromine. In yet a further embodiment, R⁶ is CF₃ or bromine. In another embodiment, R⁶ is -CF₃. In another embodiment, R⁶ is -Br.

In some embodiments, there are provided compounds of Formula I, provided that R⁶ is not chlorine, methyl or -O-C₆₋₁₀aryl.

In some embodiments, there are provided compounds of Formula I, wherein each C₁₋₆ haloalkyl is independently replaced with C₁₋₆ perfluoroalkyl. In another embodiment, there are provided compounds of Formula I, wherein each C₁₋₆ haloalkyl is independently replaced with C₁₋₃ haloalkyl. In another embodiment, there are provided compounds of Formula I, wherein each C₁₋₆ haloalkyl is independently replaced with C₁₋₃ perfluoroalkyl. In another embodiment, there are provided compounds of Formula I, wherein each C₁₋₆ haloalkyl is independently replaced with -CF₃.

In another embodiment, there are provided compounds of Formula I, wherein:
R¹ is -COOH or -C(O)OR^{a};
   wherein R⁸ is optionally substituted C₁₋₆ alkyl, wherein said alkyl substituent is selected from OH, halogen, -OC₁₋₈ alkyl and -(O(CH₂)₁₋₈)_{q}-OC₁₋₈ alkyl; and q is 1, 2, 3, 4, 5 or 6;
R² is unsubstituted C₁₋₆ alkyl or benzyl;
R³ is H or unsubstituted C₁₋₆ alkyl;
R⁴ is H or F;
R⁵ is H;
R⁶ is C₁₋₆ fluoroalkyl or bromine;
R⁷ is H;
R⁸ is H or F; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a}; wherein R^{a} is unsubstituted C₁₋₆ alkyl;
R² is unsubstituted C₁₋₆ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a}; wherein R^{a} is unsubstituted C₁₋₆ alkyl;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a}; wherein R^{a} is unsubstituted C₁₋₆ alkyl;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a};
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₆ alkyl or benzyl;
R³ is H or unsubstituted C₁₋₆ alkyl;
R⁴ is H or F;
R⁵ is H;
R⁶ is C₁₋₆ fluoroalkyl or bromine;
R⁷ H;
R⁸ is H or F; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₆ alkyl or benzyl;
R³ is H or unsubstituted C₁₋₃ alkyl;
R⁴ is H;
R⁵ is H;
R⁶ is C₁₋₆ fluoroalkyl or bromine;
R⁷ H;
R⁸ is H; and
n is 1 or 2;
or a tautomer thereof;
or pharmaceutically acceptable salt of any one of the foregoing.

In another embodiment, there are provided compounds of Formula I, wherein:
R¹ is -COOH;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ H;
R⁸ is H; and
n is 1;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ H;
R⁸ is H; and
n is 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1 or 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₃ alkyl or benzyl;
R³ is H or -CH₃;
R⁴ is H;
R⁵ is H;
R⁶ is -CF₃ or bromine;
R⁷ H;
R⁸ is H; and
n is 1;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₃ alkyl or benzyl;
R³ is H or -CH₃;
R⁴ is H;
R⁵ is H;
R⁶ is -CF₃ or bromine;
R⁷ H;
R⁸ is H; and
n is 2;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₃ alkyl or benzyl;
R³ is H;
R⁴ is H;
R⁵ is H;
R⁶ is -CF₃ or bromine;
R⁷ H;
R⁸ is H; and
n is 1;
or a single enantiomer thereof;
or a mixture of enantiomers thereof;
or a tautomer of the foregoing;
or pharmaceutically acceptable salt of the foregoing.

In another embodiment, there are provided compounds of Formula I, wherein:
R¹ is -COOH;
R² is unsubstituted C₁₋₃ alkyl or benzyl;
R³ is H;
R⁴ is H;
R⁵ is H;
R⁶ is -CF₃ or bromine;
R⁷ H;
R⁸ is H; and
n is 2;
or a single enantiomer thereof;
or a mixture of enantiomers thereof;
or a tautomer of the foregoing;
or pharmaceutically acceptable salt of the foregoing.

In some embodiments, provided herein are compounds of Formula la: 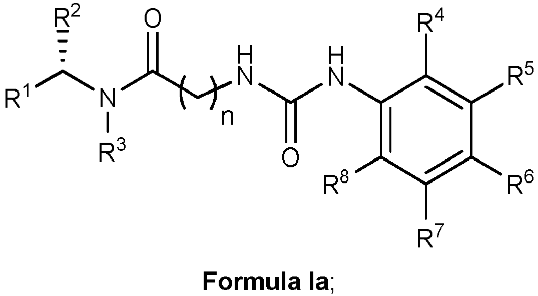 wherein the compound has the specific stereochemistry shown at the carbon bearing R², and wherein:
R¹ is -COOH or -C(O)OR^{a}; and R^{a} is optionally substituted C₁₋₆ alkyl, wherein said alkyl substituent is selected from OH, halogen, -OC₁₋₈ alkyl and -(O(CH₂)₁₋₈)_{q}-OC₁₋₈ alkyl; and q is 1, 2, 3, 4, 5 or 6;
R² is optionally substituted C₁₋₆ alkyl, wherein said optional alkyl substituent is selected from -OH, -SH, -OC₁₋₆ alkyl, -SC₁₋₆ alkyl, -COOH, -C(O)OC₁₋₆ alkyl, -C(O)NH₂, C₃₋₈ cycloalkyl, C₃₋₈ cycloalkenyl, C₆₋₁₀ aryl, and heterocycle;
R³ is H or unsubstituted C₁₋₆ alkyl;
R⁴ is H, C₁₋₆ alkyl, C₁₋₆ haloalkyl, C₃₋₈ cycloalkyl, C₃₋₈ cycloalkenyl C₆₋₁₀ aryl, heterocycle, halogen, -NR¹¹R¹², -S(O)ₘR⁹, -C(O)R¹⁰,-C(O)OC₁₋₆ alkyl, -C(O)SC₁₋₆ alkyl, -OR¹³ or -SR¹³;
R⁵ is H, C₁₋₆ alkyl, C₁₋₆ haloalkyl, halogen, -S(O)ₘR⁹ or -C(O)R¹⁰;
R⁶ is C₁₋₆ alkyl, C₁₋₆ haloalkyl, C₃₋₈ cycloalkyl, C₃₋₈ cycloalkenyl, C₆₋₁₀ aryl, heterocycle, halogen, -S(O)ₘR⁹, -C(O)R¹⁰ or -OR¹¹;
R⁷ is H, C₁₋₆ alkyl, C₁₋₆ haloalkyl, halogen, -S(O)ₘR⁹ or -C(O)R¹⁰;
R⁸ is H, C₁₋₆ alkyl, C₁₋₆ haloalkyl, C₃₋₈ cycloalkyl, C₃₋₈ cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
each R⁹ is independently -OH, C₁₋₆ alkyl or C₆₋₁₀ aryl;
each R¹⁰ is independently -OH, C₁₋₆ alkyl or C₆₋₁₀ aryl;
each R¹¹ is independently H, C₁₋₈ alkyl, C₃₋₈ cycloalkyl, C₃₋₈ cycloalkenyl, C₆₋₁₀ aryl or heterocycle;
each R¹² is independently H, C₁₋₈ alkyl, C₃₋₈ cycloalkyl, C₃₋₈ cycloalkenyl, C₆₋₁₀ aryl or heterocycle;
each R¹³ is independently H or C₁₋₈ alkyl;
each m is independently 1 or 2; and
n is 1, 2 or 3;
or a tautomer thereof;
or pharmaceutically acceptable salt of the foregoing;
provided that the compound is not:

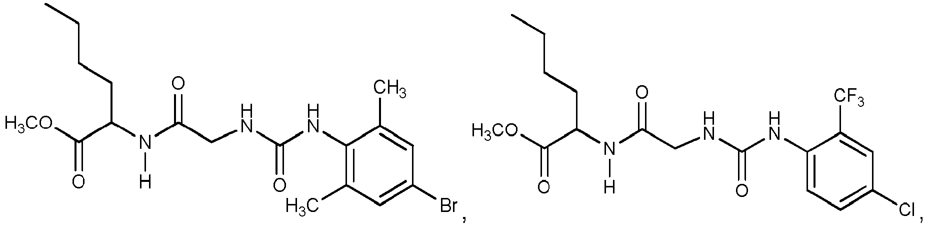

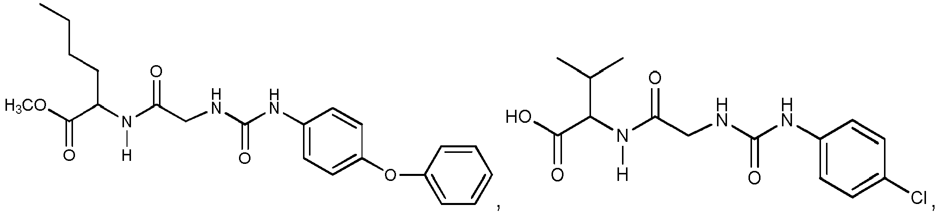

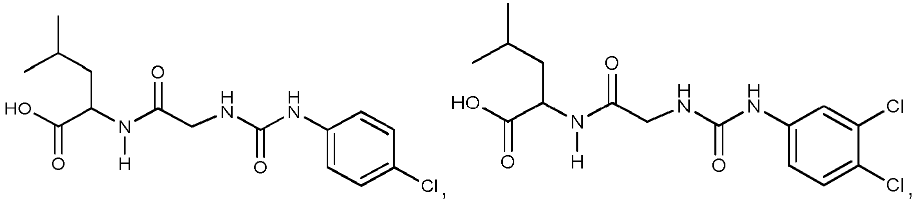

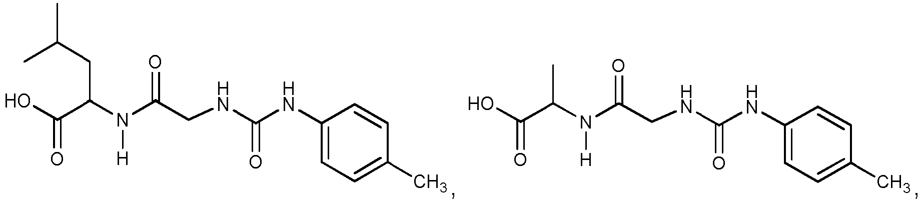

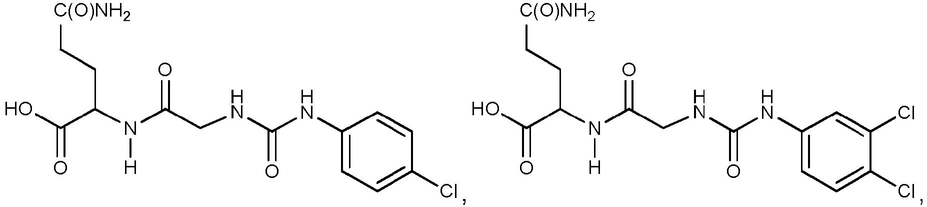

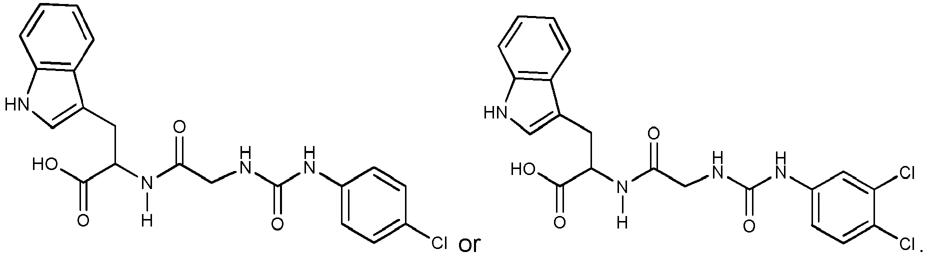

In some embodiments, there are provided compounds of Formula la, wherein R¹ is -COOH or -C(O)OR⁸, wherein R^{a} is unsubstituted or substituted C₁₋₆ alkyl; wherein said alkyl substituent is selected from OH, halogen, -OC₁₋₈ alkyl and -(O(CH₂)₁₋₈)_{q}-OC₁₋₈ alkyl; and q is 1, 2, 3, 4, 5 or 6. In further embodiments, there are provided compound of Formula la, wherein R¹ is -COOH or -C(O)OR^{a}, and R^{a} is unsubstituted C₁₋₆ alkyl. In other embodiments, there are provided compounds of Formula la, wherein R¹ is -COOH.

In some embodiments, there are provided compounds of Formula la, wherein R² is unsubstituted C₁₋₆ alkyl or benzyl. In some embodiments, R² is unsubstituted C₁₋₆ alkyl. In some embodiments, R² is unsubstituted C₁₋₄ alkyl, such as methyl, ethyl, n-propyl, isopropyl, n-butyl, tert-butyl, isobutyl or sec-butyl. In other embodiments, R² is unsubstituted benzyl.

In some embodiments, there are provided compounds of Formula la, wherein R³ is H or unsubstituted C₁₋₃ alkyl. In some embodiments, R³ is H. In another embodiment, R³ is unsubstituted C₁₋₃ alkyl, such as methyl, ethyl, n-propyl or isopropyl. In yet another embodiment, R³ is methyl.

In some embodiments, there are provided compounds of Formula la, wherein n is 1 or 2. In another embodiment, n is 1. In yet other embodiment, n is 2.

In some embodiments, there are provided compounds of Formula la, wherein R⁴ is H, F or C₁₋₆ haloalkyl; R⁵ is H, F or C₁₋₆ haloalkyl; R⁷ is H, F or C₁₋₆ haloalkyl; and R⁸ is H, F or C₁₋₆ haloalkyl. In another embodiment, there are provided compounds wherein R⁴ is H, F or C₁₋₆ fluoroalkyl; R⁵ is H, F or C₁₋₆ fluoroalkyl; R⁷ is H, F or C₁₋₆ fluoroalkyl; and R⁸ is H, F or C₁₋₆ fluoroalkyl. In a further embodiment, there are provided compounds of Formula Ia wherein R⁴ is H, F or C₁₋₆ perfluoroalkyl; R⁵ is H, F or C₁₋₆ perfluoroalkyl; R⁷ is H, F or C₁₋₆ perfluoroalkyl; and R⁸ is H, F or C₁₋₆ perfluoroalkyl. In yet a further embodiment, there are provided compounds of Formula Ia wherein R⁴ is H, F or CF₃; R⁵ is H, F or CF₃; R⁷ is H, F or CF₃; and R⁸ is H, F or CF₃. In another embodiment, R⁴ is H or F; R⁵ is H or F; R⁷ is H or F; and R⁸ is H or F. In a further embodiment, there are provided compounds of Formula la, wherein at least one of R⁴ and R⁸ is H. In other embodiment, there are provided compounds of Formula la, wherein each of R⁴, R⁵, R⁷ and R⁸ is H.

In some embodiments, there are provided compounds of Formula la, wherein R⁶ is C₁₋₆ haloalkyl or halogen. In one embodiment, R⁶ is C₁₋₆ haloalkyl or Br. In another embodiment, R⁶ is C₁₋₆ fluoroalkyl or bromine. In a further embodiment, R⁶ is C₁₋₆ perfluoroalkyl or bromine. In yet a further embodiment, R⁶ is CF₃ or bromine. In another embodiment, R⁶ is -CF₃. In another embodiment, R⁶ is -Br.

In some embodiments, there are provided compounds of Formula la, provided that R⁶ is not chlorine, methyl or -O-C₆₋₁₀aryl.

In some embodiments, there are provided compounds of Formula la, wherein each C₁₋₆ haloalkyl is independently replaced with C₁₋₆ perfluoroalkyl. In another embodiment, there are provided compounds of Formula la, wherein each C₁₋₆ haloalkyl is independently replaced with C₁₋₃ haloalkyl. In another embodiment, there are provided compounds of Formula la, wherein each C₁₋₆ haloalkyl is independently replaced with C₁₋₃ perfluoroalkyl. In another embodiment, there are provided compounds of Formula la, wherein each C₁₋₆ haloalkyl is independently replaced with -CF₃.

In another embodiment, there are provided compounds of Formula la, wherein:
R¹ is -COOH or -C(O)OR^{a};
   wherein R^{a} is optionally substituted C₁₋₆ alkyl, wherein said alkyl substituent is selected from OH, halogen, -OC₁₋₈ alkyl and -(O(CH₂)₁₋₈)_{q}-OC₁₋₈ alkyl; and q is 1, 2, 3, 4, 5 or 6;
R² is unsubstituted C₁₋₆ alkyl or benzyl;
R³ is H or unsubstituted C₁₋₆ alkyl;
R⁴ is H or F;
R⁵ is H;
R⁶ is C₁₋₆ fluoroalkyl or bromine;
R⁷ is H;
R⁸ is H or F; and
n is 1 or 2;
or a tautomer thereof;
or pharmaceutically acceptable salt of the foregoing.

In another embodiment, there are provided compounds of Formula la, wherein:
R¹ is -COOH or -C(O)OR^{a}; wherein R^{a} is unsubstituted C₁₋₆ alkyl;
R² is unsubstituted C₁₋₆ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1 or 2;
or a tautomer thereof;
or pharmaceutically acceptable salt of any one of the foregoing.

In another embodiment, there are provided compounds of Formula la, wherein:
R¹ is -COOH or -C(O)OR^{a}; wherein R^{a} is unsubstituted C₁₋₆ alkyl;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1 or 2;
or a tautomer thereof;
or pharmaceutically acceptable salt of the foregoing.

In another embodiment, there are provided compounds of Formula la, wherein:
R¹ is -COOH or -C(O)OR^{a}; wherein R^{a} is unsubstituted C₁₋₆ alkyl;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1;
or a tautomer thereof;
or pharmaceutically acceptable salt of the foregoing.

In another embodiment, there are provided compounds of Formula la, wherein:
R¹ is -COOH or -C(O)OR^{a}; wherein R^{a} is unsubstituted C₁₋₆ alkyl;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 2;
or a tautomer thereof;
or pharmaceutically acceptable salt of the foregoing.

In another embodiment, there are provided compounds of Formula la, wherein:
R¹ is -COOH;
R² is unsubstituted C₁₋₆ alkyl or benzyl;
R³ is H or unsubstituted C₁₋₆ alkyl;
R⁴ is H or F;
R⁵ is H;
R⁶ is C₁₋₆ fluoroalkyl or bromine;
R⁷ H;
R⁸ is H or F; and
n is 1 or 2;
or a tautomer thereof;
or pharmaceutically acceptable salt of the foregoing.

In another embodiment, there are provided compounds of Formula la, wherein:
R¹ is -COOH;
R² is unsubstituted C₁₋₆ alkyl or benzyl;
R³ is H or unsubstituted C₁₋₃ alkyl;
R⁴ is H;
R⁵ is H;
R⁶ is C₁₋₆ fluoroalkyl or bromine;
R⁷ H;
R⁸ is H; and
n is 1 or 2;
or a tautomer thereof;
or pharmaceutically acceptable salt of any one of the foregoing.

In another embodiment, there are provided compounds of Formula la, wherein:
R¹ is -COOH;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ H;
R⁸ is H; and
n is 1 or 2;
or a tautomer thereof;
or pharmaceutically acceptable salt of the foregoing.

In another embodiment, there are provided compounds of Formula la, wherein:
R¹ is -COOH;
R² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 1;
or a tautomer thereof;
or pharmaceutically acceptable salt of the foregoing.

In another embodiment, there are provided compounds of Formula la, wherein:
R¹ is -COOHR² is unsubstituted C₁₋₄ alkyl or benzyl;
R³ is H or unsubstituted C₁₋₃ alkyl;
R⁴ is H;
R⁵ is H;
R⁶ is C₁₋₆ fluoroalkyl or bromine;
R⁷ is H;
R⁸ is H; and
n is 2;
or a tautomer thereof;
or pharmaceutically acceptable salt of the foregoing.

In another embodiment, there are provided compounds of Formula la, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1 or 2;
or a tautomer thereof;
or pharmaceutically acceptable salt of the foregoing.

In another embodiment, there are provided compounds of Formula la, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1;
or a tautomer thereof;
or pharmaceutically acceptable salt of the foregoing.

In another embodiment, there are provided compounds of Formula la, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 2;
or a tautomer thereof;
or pharmaceutically acceptable salt of the foregoing.

In another embodiment, there are provided compounds of Formula la, wherein:
R¹ is -COOH;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1 or 2;
or a tautomer thereof;
or pharmaceutically acceptable salt of the foregoing.

In another embodiment, there are provided compounds of Formula la, wherein:
R¹ is -COOH;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1;
or a tautomer thereof;
or pharmaceutically acceptable salt of the foregoing.

In another embodiment, there are provided compounds of Formula la, wherein:
R¹ is -COOH;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 2;
or a tautomer thereof;
or pharmaceutically acceptable salt of the foregoing.

In another embodiment, there are provided compounds of Formula la, wherein:
R¹ is -COOH;
R² is unsubstituted C₁₋₃ alkyl or benzyl;
R³ is H;
R⁴ is H;
R⁵ is H;
R⁶ is -CF₃ or bromine;
R⁷ is H;
R⁸ is H; and
n is 1;
or a tautomer thereof;
or pharmaceutically acceptable salt of the foregoing.

In another embodiment, there are provided compounds of Formula la, wherein:
R¹ is -COOH;
R² is unsubstituted C₁₋₃ alkyl or benzyl;
R³ is H;
R⁴ is H;
R⁵ is H;
R⁶ is -CF₃ or bromine;
R⁷ is H;
R⁸ is H; and
n is 2;
or a tautomer thereof;
or pharmaceutically acceptable salt of the foregoing.

In some embodiments, the invention provides for a compound selected from:
(S)-2-(2-(3-(4-bromophenyl)ureido)acetamido)-4-methylpentanoic acid;
(S)-4-methyl-2-(2-(3-(4-(trifluoromethyl)phenyl)ureido)acetamido)pentanoic acid;
(S)-2-(2-(3-(4-bromophenyl)ureido)acetamido)-3-methylbutanoic acid;
(S)-2-(2-(3-(4-bromophenyl)ureido)acetamido)-3-phenylpropanoic acid;
(S)-2-(3-(3-(4-bromophenyl)ureido)propanamido)-4-methylpentanoic acid;
(S)-2-(2-(3-(4-bromophenyl)ureido)acetamido)pentanoic acid;
(S)-2-(2-(3-(4-(trifluoromethyl)phenyl)ureido)acetamido)pentanoic acid;
(S)-tert-butyl 2-(2-(3-(4-bromophenyl)ureido)acetamido)pentanoate; and
(S)-tert-butyl 2-(2-(3-(4-(trifluoromethyl)phenyl)ureido)acetamido) pentanoate;
and pharmaceutically acceptable salts thereof.

In some embodiments, the invention provides for a compound selected from: 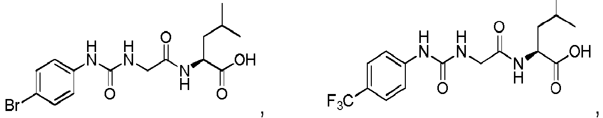 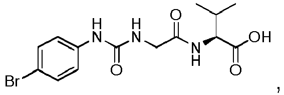 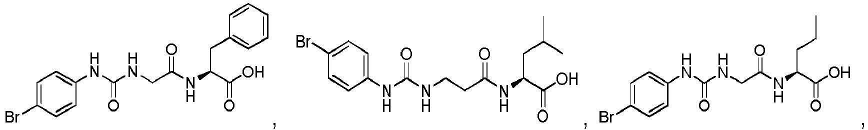 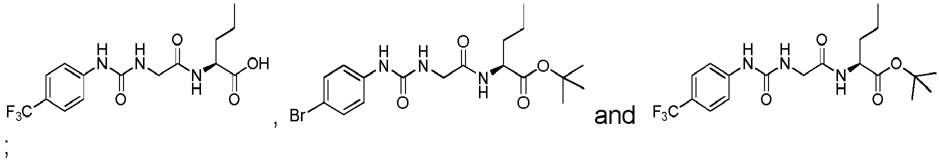 and pharmaceutically acceptable salts thereof.

In some embodiments, the invention provides for a compound selected from: 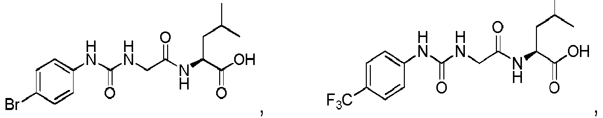 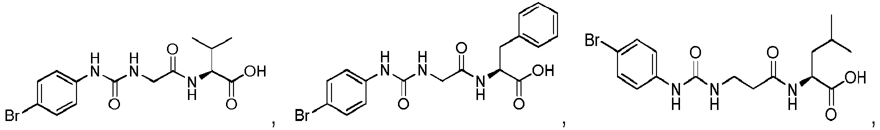 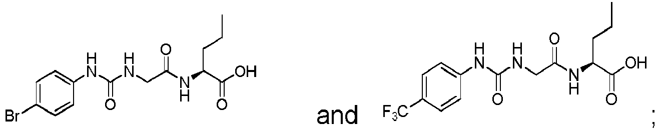 and pharmaceutically acceptable salts thereof.

Some compounds of the invention may form salts with acids or bases, including pharmaceutically acceptable acids or bases. Such pharmaceutically acceptable salts of the compounds described herein are within the scope of the invention.

The acid addition salt form of a compound of Formula I or la that occurs in its free form as a base can be obtained by treating the free base with an appropriate acid such as an inorganic acid, for example, hydrochloric acid, hydrobromic acid, sulfuric acid, phosphoric acid, nitric acid and the like; or an organic acid such as for example, acetic acid, hydroxyacetic acid, propanoic acid, lactic acid, pyruvic acid, malonic acid, fumaric acid, maleic acid, oxalic acid, tartaric acid, succinic acid, malic acid, ascorbic acid, benzoic acid, tannic acid, pamoic acid, citric acid, methylsulfonic acid, ethanesulfonic acid, benzenesulfonic acid, formic acid and the like. The base addition salt form of a compound of Formula I or la that occurs in its acid form can be obtained by treating the acid with an appropriate base such as an inorganic base, for example, sodium hydroxide, magnesium hydroxide, potassium hydroxide, calcium hydroxide, ammonia and the like; or an organic base such as for example, L-arginine, ethanolamine, betaine, benzathine, morpholine and the like. (See Handbook of Pharmaceutical Salts, P. Heinrich Stahl & Camille G. Wermuth (Eds), Verlag Helvetica Chimica Acta, Zurich, 2002, 329-345.)

Some of the compounds of Formula I or la and some of their intermediates may contain one or more asymmetric centers in their structure; each asymmetric center may be present in an *R* or *S* configuration, said *R* and *S* notation corresponding to the rules described in Pure and Applied Chemistry (1976), 45, 11-13. As such, the compounds may exist in enantiomeric as well as in diastereomeric forms. Unless it is specifically noted otherwise, the scope of the present invention includes all enantiomers, diastereomers and mixtures thereof, including racemic mixtures.

As will be evident to those skilled in the art, individual diastereoisomeric forms can be obtained by separation of mixtures thereof in a conventional manner. For example, chromatographic separation may be employed; chiral chromatography may be performed to separate individual enantiomers.

In an embodiment of the invention, there are provided pharmaceutical compositions including a therapeutically effective amount of at least one compound of the invention in a pharmaceutically acceptable carrier.

The compounds of the invention and the pharmaceutical compositions comprising at least one compound of the invention are indicated for use in treating or preventing conditions in which there is likely to be a component involving the FPR, such as FPR1 and/or FPR2.

More specifically, the present invention provides for:
use of a compound of the invention in the manufacture of a medicament for the treatment of a mammalian subject, including a human subject, having one or more diseases or conditions that are alleviated by FPR modulation (such as FPR1 and/or FPR2 agonism, or selective agonism of FPR1 relative to FPR2);
wherein the disease or condition is an ocular disease or condition, including but not limited to: ocular inflammation, age-related macular degeneration, wet macular degeneration, dry macular degeneration, uveitis, dry eye, keratitis, allergic eye disease and conditions affecting the posterior part of the eye, such as maculopathies and retinal degeneration including non-exudative age related macular degeneration, exudative age related macular degeneration, choroidal neovascularization, diabetic retinopathy (proliferative), retinopathy of prematurity, acute macular neuroretinopathy, central serous chorioretinopathy, cystoid macular edema, and diabetic macular edema; infectious keratitis, herpetic keratitis, corneal angiogenesis, lymphangiogenesis, uveitis, retinitis, choroiditis, such as acute multifocal placoid pigment epitheliopathy, Behcet's disease, birdshot retinochoroidopathy, infectious (syphilis, lyme, tuberculosis, toxoplasmosis), intermediate uveitis (pars planitis), multifocal choroiditis, multiple evanescent white dot syndrome (mewds), ocular sarcoidosis, posterior scleritis, serpiginous choroiditis, subretinal fibrosis and uveitis syndrome, Vogt-Koyanagi-and Harada syndrome; vascular diseases/exudative diseases such as retinal arterial occlusive disease, central retinal vein occlusion, cystoids macular edema, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angiitis, sickle cell retinopathy and other hemoglobinopathies, angioid streaks, familial exudative vitreoretinopathy, and Eales disease; traumatic/surgical conditions such as sympathetic ophthalmia, uveitic retinal disease, retinal detachment, trauma, conditions caused by laser, conditions caused by photodynamic therapy, photocoagulation, hypoperfusion during surgery, radiation retinopathy, bone marrow transplant retinopathy, corneal wound healing, post-surgical corneal wound healing and/or inflammation, and post-cataract surgical inflammation; proliferative disorders such as proliferative vitreal retinopathy and epiretinal membranes, and proliferative diabetic retinopathy; infectious disorders such as ocular histoplasmosis, ocular toxocariasis, presumed ocular histoplasmosis syndrome (POHS), endophthalmitis, toxoplasmosis, retinal diseases associated with HIV infection, choroidal disease associate with HIV infection, uveitic disease associate with HIV infection, viral retinitis, acute retinal necrosis, progressive outer retinal necrosis, fungal retinal diseases, ocular syphilis, ocular tuberculosis, diffuse unilateral subacute neuroretinitis, and myiasis; genetic disorders such as retinitis pigmentosa, systemic disorders with associated retinal dystrophies, congenital stationary night blindness, cone dystrophies, Stargardt's disease and fundus flavimaculatus, Best's disease, pattern dystrophy of the retinal pigmented epithelium, X-linked retinoschisis, Sorsby's fundus dystrophy, benign concentric maculopathy, Bietti's crystalline dystrophy, and pseudoxanthoma elasticum; retinal tears/holes such as retinal detachment, macular hole, and giant retinal tear; tumors such as retinal disease associated with tumors, congenital hypertrophy of the retinal pigmented epithelium, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, and intraocular lymphoid tumors; and miscellaneous other diseases affecting the posterior part of the eye such as punctate inner choroidopathy, acute posterior multifocal placoid pigment epitheliopathy, myopic retinal degeneration, and acute retinal pigment epitheliitis, blepharitis, meibomian gland dysfunction (MDG), glaucoma, branch vein occlusion, Best's vitelliform macular degeneration, retinitis pigmentosa, proliferative vitreoretinopathy (PVR), and any other degenerative disease of either the photoreceptors or the retinal pigment epithelium (Perretti, Mauro et al. Pharmacology & Therapeutics 127 (2010) 175-188).

In other embodiments, the present invention provides for a compound or composition of the invention for use in a method of treating a mammalian subject, including a human subject, having one or more diseases or conditions that are alleviated by FPR modulation (such as FPR1 and/or FPR2 agonism, or selective agonism of FPR1 relative to FPR2);
wherein the disease or condition is a dermal disease or condition, including, but not limited to: dermal inflammation, dermal wound healing, hypertrophic scars, keloids, burns, sunburn, rosacea, erythema of the skin, atopic dermatitis, acne, psoriasis, seborrheic dermatitis, actinic keratoses, basal cell carcinoma, squamous cell carcinoma, melanoma, viral warts, photoaging, photodamage, melasma, post-inflammatory hyperpigmentation, disorders of pigmentation, alopecia, scarring and non-scarring forms.

In yet other embodiments, the present invention provides for a compound or composition of the invention for use in a method of treating a mammalian subject, including a human subject, having one or more diseases or conditions that are alleviated by FPR modulation (such as FPR1 and/or FPR2 agonism, or selective agonism of FPR1 relative to FPR2);
wherein the disease or condition is selected from: a systemic inflammatory disease or condition, stroke, coronary artery disease, a cardiovascular disorder, coronary artery disease, angina pectoris; or
an obstructive airway disease; or
a neurological disorder, a central nervous system disorder, Alzheimer's disease, neuroinflammation or pain; or
an HIV-mediated retroviral infection; or
an immunological disorder, arthritis, rheumatoid arthritis, systemic lupus erythematosus, multiple sclerosis; or
sepsis; or
inflammatory bowel disease or ulcerative colitis; or
asthma or an allergic disorder; or
cachexia.

In a further embodiment of the invention, there is provided a compound or composition of the invention for use in the method of treating a disease or condition alleviated by FPR modulation (such as FPR1 and/or FPR2 agonism, or selective agonism of FPR1 relative to FPR2), wherein the method comprises administering to the subject in need of the treatment a therapeutically effective amount of at least one compound of the invention, or an enantiomer, diastereomer or tautomer thereof, or pharmaceutically acceptable salt of any one of the foregoing.

In one embodiment, the invention provides a compound or composition of the invention for use in for a method of treating a disease or condition in a subject in need of such treatment, the method comprising administering a therapeutically effective amount of a compound of Formula I or la to the subject, thereby treating the disease or condition. In one embodiment, the method comprises administering a compound of Formula I. In another embodiment, the method comprises administering a compound of Formula Ia.

In another embodiment, the invention provides for a compound or composition of the invention for use in a method of treating a disease or condition in a subject in need of such treatment, the method comprising administering a pharmaceutical composition comprising a therapeutically effective amount of a compound of Formula I or Ia to the subject, thereby treating the disease or condition. In one embodiment, the method comprises administering a pharmaceutical composition comprising a therapeutically effective amount of a compound of Formula I. In one embodiment, the method comprises administering a pharmaceutical composition comprising a therapeutically effective amount of a compound of Formula Ia.

In another embodiment, there is provided a compound or composition of the invention for use in a method of treating the disease or condition associated with FPR modulation (such as FPR1 and/or FPR2 agonism, or selective agonism of FPR1 relative to FPR2) in a subject in need of such treatment, wherein the disease or condition is an ocular disease or condition; the method comprising administering a pharmaceutical composition comprising a therapeutically effective amount of a compound of Formula I or Ia to the subject, thereby treating the disease or condition. In a further embodiment, the ocular disease or condition selected from: ocular inflammation, age-related macular degeneration, wet macular degeneration, dry macular degeneration, uveitis, dry eye, keratitis, allergic eye disease and conditions affecting the posterior part of the eye, such as maculopathies and retinal degeneration including non-exudative age related macular degeneration, exudative age related macular degeneration, choroidal neovascularization, diabetic retinopathy (proliferative), retinopathy of prematurity, acute macular neuroretinopathy, central serous chorioretinopathy, cystoid macular edema, and diabetic macular edema; infectious keratitis, herpetic keratitis, corneal angiogenesis, lymphangiogenesis, retinitis, and choroiditis such as acute multifocal placoid pigment epitheliopathy, Behcet's disease, birdshot retinochoroidopathy, infectious (syphilis, lyme, tuberculosis, toxoplasmosis), intermediate uveitis (pars planitis), multifocal choroiditis, multiple evanescent white dot syndrome (mewds), ocular sarcoidosis, posterior scleritis, serpiginous choroiditis, subretinal fibrosis and uveitis syndrome, Vogt-Koyanagi-and Harada syndrome; vascular diseases/ exudative diseases such as retinal arterial occlusive disease, central retinal vein occlusion, cystoids macular edema, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angiitis, sickle cell retinopathy and other hemoglobinopathies, angioid streaks, familial exudative vitreoretinopathy, and Eales disease; traumatic/surgical conditions such as sympathetic ophthalmia, uveitic retinal disease, retinal detachment, trauma, conditions caused by laser, conditions caused by photodynamic therapy, photocoagulation, hypoperfusion during surgery, radiation retinopathy, bone marrow transplant retinopathy, post-surgical corneal wound healing or inflammation, and post-cataract surgical inflammation; proliferative disorders such as proliferative vitreal retinopathy and epiretinal membranes, and proliferative diabetic retinopathy; infectious disorders such as ocular histoplasmosis, ocular toxocariasis, presumed ocular histoplasmosis syndrome (POHS), endophthalmitis, toxoplasmosis, retinal diseases associated with HIV infection, choroidal disease associate with HIV infection, uveitic disease associate with HIV infection, viral retinitis, acute retinal necrosis, progressive outer retinal necrosis, fungal retinal diseases, ocular syphilis, ocular tuberculosis, diffuse unilateral subacute neuroretinitis, and myiasis; genetic disorders such as retinitis pigmentosa, systemic disorders with associated retinal dystrophies, congenital stationary night blindness, cone dystrophies, Stargardt's disease and fundus flavimaculatus, Best's disease, pattern dystrophy of the retinal pigmented epithelium, X-linked retinoschisis, Sorsby's fundus dystrophy, benign concentric maculopathy, Bietti's crystalline dystrophy, and pseudoxanthoma elasticum; retinal tears/holes such as retinal detachment, macular hole, and giant retinal tear; tumors such as retinal disease associated with tumors, congenital hypertrophy of the retinal pigmented epithelium, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, and intraocular lymphoid tumors; and miscellaneous other diseases affecting the posterior part of the eye such as punctate inner choroidopathy, acute posterior multifocal placoid pigment epitheliopathy, myopic retinal degeneration, and acute retinal pigment epitheliitis, post-surgical corneal inflammation, blepharitis, meibomian gland dysfunction (MGD), corneal wound healing, glaucoma, branch vein occlusion, Best's vitelliform macular degeneration, retinitis pigmentosa, proliferative vitreoretinopathy (PVR), and any other degenerative disease of either the photoreceptors or the retinal pigment epithelium. In a further embodiment, the ocular inflammatory disease or condition is selected from: dry eye, a post-surgical corneal wound, post-surgical corneal inflammation, and post-cataract surgical inflammation. In a further embodiment, the subject is a human.

In another embodiment, there is provided a compound or composition of the invention for use in a method of treating the disease or condition associated with FPR modulation (such as FPR1 and/or FPR2 agonism, or selective agonism of FPR1 relative to FPR2) in a subject in need of such treatment, wherein the disease or condition is a dermal disease or condition; the method comprising administering a pharmaceutical composition comprising a therapeutically effective amount of a compound of Formula I or Ia to the subject, thereby treating the disease or condition. In a further embodiment, the dermal disease or condition is selected from: dermal inflammation, a dermal wound, hypertrophic scars, keloids, burns, sunburn, rosacea, erythema of the skin, atopic dermatitis, acne, psoriasis, seborrheic dermatitis, actinic keratoses, basal cell carcinoma, squamous cell carcinoma, melanoma, viral warts, photoaging, photodamage, melasma, post-inflammatory hyperpigmentation, disorders of pigmentation, alopecia, scarring and non-scarring forms. In a further embodiment, the dermal disease or condition is psoriasis or rosacea. In a further embodiment, the subject is a human.

In another embodiment, there is provided a compound or composition of the invention for use in the method of treating the disease or condition associated with FPR modulation (such as FPR1 and/or FPR2 agonism, or FPR1 agonism, or selective agonism of FPR1 relative to FPR2) in a subject in need of such treatment, wherein the disease or condition is a systemic inflammatory condition, stroke, coronary artery disease, a cardiovascular disorder, coronary artery disease or angina pectoris; or an obstructive airway disease; or a neurological disorder, Alzheimer's disease, neuroinflammation or pain; or an HIV-mediated retroviral infection; or an immunological disorder, arthritis, rheumatoid arthritis, systemic lupus erythematosus, multiple sclerosis; or sepsis; or inflammatory bowel disease or ulcerative colitis; or asthma or an allergic disorder; or cachexia; the method comprising administering a pharmaceutical composition comprising a therapeutically effective amount of a compound of Formula I or Ia to the subject, thereby treating the disease or condition. In a further embodiment, the disease or condition is rheumatoid arthritis. In one embodiment, the disease or condition is multiple sclerosis. In another embodiment, the disease or condition is inflammatory bowel disease. In one embodiment, the disease or condition is ulcerative colitis. In a further embodiment, the condition is pain. In a further embodiment, the subject is a human.

The actual amount of the compound to be administered in any given case will be determined by a physician taking into account the relevant circumstances, such as the severity of the condition, the age and weight of the subject/patient, the patient's general physical condition, the cause of the condition, and the route of administration.

The subject will be administered the compound orally in any acceptable form, such as a tablet, liquid, capsule, powder and the like, or other routes may be desirable or necessary, particularly if the patient suffers from nausea. Such other routes may include, without exception, transdermal, parenteral, subcutaneous, intranasal, via an implant stent, intrathecal, intravitreal, topical to the eye, back of the eye, intramuscular, intravenous, and intrarectal modes of delivery. Additionally, the formulations may be designed to delay release of the active compound over a given period of time, or to carefully control the amount of drug released at a given time during the course of therapy.

In another embodiment of the invention, there are provided pharmaceutical compositions including at least one compound of the invention in a pharmaceutically acceptable carrier thereof. The phrase "pharmaceutically acceptable" means the carrier, diluent or excipient must be compatible with the other ingredients of the formulation and not deleterious to the recipient thereof.

Pharmaceutical compositions of the present invention can be used in the form of a solid, a solution, an emulsion, a dispersion, a patch, a micelle, a liposome, and the like, wherein the resulting composition contains one or more compounds of the present invention, as an active ingredient, in admixture with an organic or inorganic carrier or excipient suitable for enteral or parenteral applications. Invention compounds may be combined, for example, with the usual non-toxic, pharmaceutically acceptable carriers for tablets, pellets, capsules, suppositories, solutions, emulsions, suspensions, and any other form suitable for use. The carriers which can be used include glucose, lactose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition, auxiliary, stabilizing, thickening and coloring agents and perfumes may be used. Invention compounds are included in the pharmaceutical composition in an amount sufficient to produce the desired effect upon the process or disease condition.

Pharmaceutical compositions containing invention compounds may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of a sweetening agent such as sucrose, lactose, or saccharin, flavoring agents such as peppermint, oil of wintergreen or cherry, coloring agents and preserving agents in order to provide pharmaceutically elegant and palatable preparations. Tablets containing invention compounds in admixture with non-toxic pharmaceutically acceptable excipients may also be manufactured by known methods. The excipients used may be, for example, (1) inert diluents such as calcium carbonate, lactose, calcium phosphate or sodium phosphate; (2) granulating and disintegrating agents such as corn starch, potato starch or alginic acid; (3) binding agents such as gum tragacanth, corn starch, gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

In some cases, formulations for oral use may be in the form of hard gelatin capsules wherein the invention compounds are mixed with an inert solid diluent, for example, calcium carbonate, calcium phosphate or kaolin. They may also be in the form of soft gelatin capsules wherein the invention compounds are mixed with water or an oil medium, for example, peanut oil, liquid paraffin or olive oil.

The pharmaceutical compositions may be in the form of a sterile injectable suspension. This suspension may be formulated according to known methods using suitable dispersing or wetting agents and suspending agents. The sterile injectable preparation may also be a sterile injectable solution or suspension in a non-toxic parenterally-acceptable diluent or solvent, for example, as a solution in 1,3-butanediol. Sterile, fixed oils are conventionally employed as a solvent or suspending medium. For this purpose, any bland fixed oil may be employed, including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like. Buffers, preservatives, antioxidants, and the like can be incorporated as required.

Pharmaceutical compositions containing invention compounds may be in a form suitable for topical use, for example, as oily suspensions, as solutions or suspensions in aqueous liquids or nonaqueous liquids, or as oil-in-water or water-in-oil liquid emulsions. Pharmaceutical compositions may be prepared by combining a therapeutically effective amount of at least one compound according to the present invention, or a pharmaceutically acceptable salt thereof, as an active ingredient with conventional ophthalmically acceptable pharmaceutical excipients and by preparation of unit dosage suitable for topical ocular use. The therapeutically efficient amount typically is between about 0.001 and about 5% (w/v), preferably about 0.001 to about 2.0% (w/v) in liquid formulations.

For ophthalmic application, preferably solutions are prepared using a physiological saline solution as a major vehicle. The pH of such ophthalmic solutions should preferably be maintained between 4.5 and 8.0 with an appropriate buffer system, a neutral pH being preferred but not essential. The formulations may also contain conventional pharmaceutically acceptable preservatives, stabilizers and surfactants. Preferred preservatives that may be used in the pharmaceutical compositions of the present invention include, but are not limited to, benzalkonium chloride, chlorobutanol, thimerosal, phenylmercuric acetate and phenylmercuric nitrate. A preferred surfactant is, for example, Tween 80. Likewise, various preferred vehicles may be used in the ophthalmic preparations of the present invention. These vehicles include, but are not limited to, polyvinyl alcohol, povidone, hydroxypropyl methyl cellulose, poloxamers, carboxymethyl cellulose, hydroxyethyl cellulose cyclodextrin and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar manner an ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place of or in conjunction with it.

The ingredients are usually used in the following amounts:

| **Ingredient** | **Amount (% w/v)** |
|---|---|
| active ingredient | about 0.001-5 |
| preservative | 0-0.10 |
| vehicle | 0-40 |
| tonicity adjustor | 0-10 |
| buffer | 0.01-10 |
| pH adjustor | q.s. pH 4.5-7.8 |
| antioxidant | as needed |
| surfactant | as needed |
| purified water | to make 100% |

The actual dose of the active compounds of the present invention depends on the specific compound, and on the condition to be treated; the selection of the appropriate dose is well within the knowledge of the skilled artisan.

The ophthalmic formulations of the present invention are conveniently packaged in forms suitable for metered application, such as in containers equipped with a dropper, to facilitate application to the eye. Containers suitable for dropwise application are usually made of suitable inert, non-toxic plastic material, and generally contain between about 0.5 and about 15 ml solution. One package may contain one or more unit doses. Preservative-free solutions are often formulated in non-resalable containers containing up to about ten, preferably up to about five units doses, where a typical unit dose is from one to about 8 drops, preferably one to about 3 drops. The volume of one drop usually is about 20-35 microliters.

The compounds of the invention may also be administered in the form of suppositories for rectal administration of the drug. These compositions may be prepared by mixing the invention compounds with a suitable non-irritating excipient, such as cocoa butter, synthetic glyceride esters of polyethylene glycols, which are solid at ordinary temperatures, but liquefy and/or dissolve in the rectal cavity to release the drug.

Since individual subjects may present a wide variation in severity of symptoms and each drug has its unique therapeutic characteristics, the precise mode of administration and dosage employed for each subject is left to the discretion of the practitioner.

In one embodiment, the invention provides for a pharmaceutical composition comprising as active ingredient a therapeutically effective amount of a compound of Formula I or la, and a pharmaceutically acceptable carrier. In a further embodiment, there is provided a compound of Formula I or Ia or pharmaceutical composition comprising a compound of Formula I or Ia or any compound specifically set forth herein, for use in treating an inflammatory disease or condition in a subject in need of such treatment, wherein the disease or condition is an ocular inflammatory disease or condition, a dermal inflammatory disease or condition, a systemic inflammatory disease or condition, or an autoimmune disease or condition.

The present disclosure concerns also processes for preparing the compounds of Formula I and Ia. Synthetic Scheme 1a, set forth below, illustrates how the compounds according to the invention can be made. Further examples are provided in Scheme 1b.

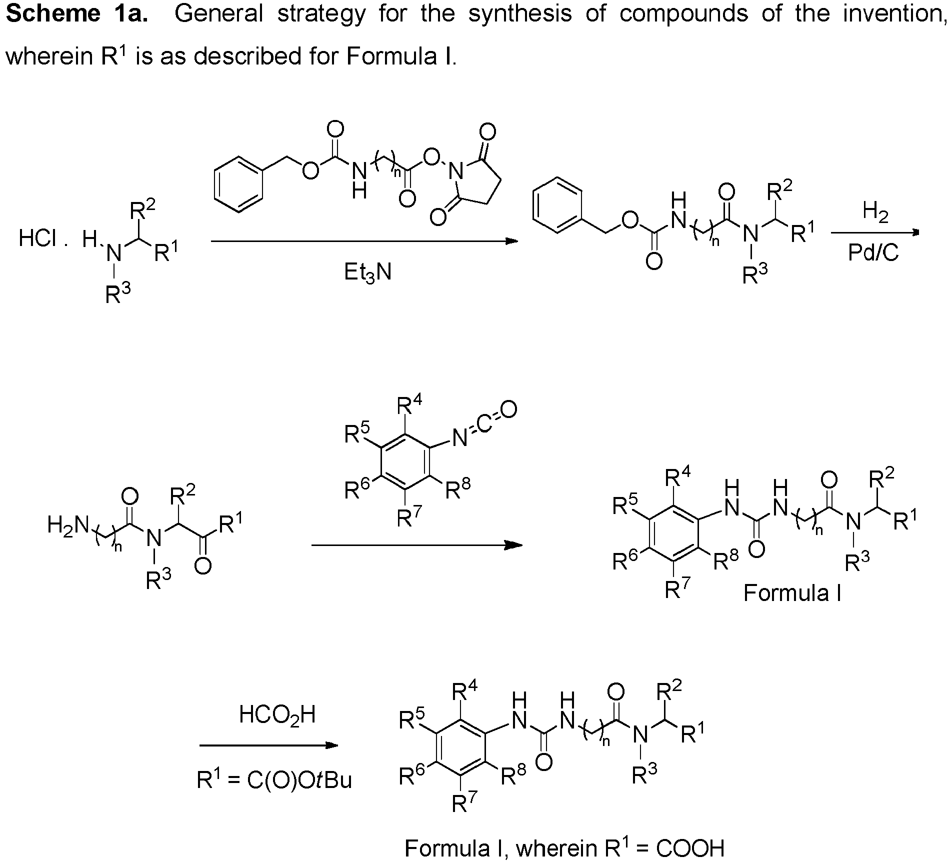

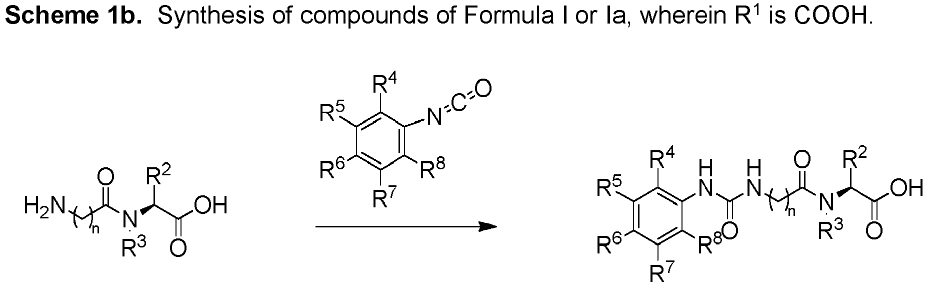

Those skilled in the art will be able to routinely modify and/or adapt Scheme 1a and/or 1b to synthesize any compounds of the invention that fall within the scope of Formula I or Ia.

The following Examples illustrate how compounds according to the invention can be made, and provide details of certain specific chemical transformations. The Examples are for illustrative purposes only and are not intended, nor should they be construed, as limiting the invention in any manner. Those skilled in the art will be able to routinely modify and/or adapt the Examples to synthesize any compound of the invention covered by Formula I and la, and will appreciate that variations and modifications of the Examples can be made without exceeding the scope of the invention.

The following abbreviations are used herein:
- CD₃OD: deuterated methanol
- Et₃N: triethylamine
- EtOAc: ethyl acetate
- H₂: hydrogen gas
- HCO₂H: formic acid
- Na₂SO₄: sodium sulfate
- Pd/C: palladium on carbon
- THF: tertahydrofuran
- TMS: tetramethylsilane

All reagents, solvents and catalysts for which the synthesis is not described are purchased from chemical vendors such as 3B Scientific, Sigma Aldrich, Fluka, Bio-Blocks, Combi-blocks, TCI, VWR, Lancaster, Oakwood, Trans World Chemical, Alfa, Fisher, Maybridge, Frontier, Matrix, Ukrorgsynth, Toronto, Ryan Scientific, SiliCycle, Anaspec, Syn Chem, Chem-Impex, MIC-scientific, Ltd; however some known intermediates were prepared according to published procedures.

Compound names were generated with ACDLab version 12.5; some intermediate and reagent names used in the Examples were generated with software such as Chem Bio Draw Ultra version 12.0, ACDLab version 12.5 or Auto Nom 2000 from MDL ISIS Draw 2.5 SP1.

In general, characterization of the compounds was performed using NMR spectroscopy. NMR spectra were acquired on a 300 or 600 MHz Varian NMR spectrometer and at room temperature. Chemical shifts are given in ppm referenced either to internal TMS or to the solvent signal.

Usually, the compounds of the invention were purified by medium pressure liquid chromatography, unless noted otherwise.

### Example A

### Intermediate 1

### (S)-tert-butyl 2-(2(((benzyloxy)carbonyl)amino)acetamido)pentanoate

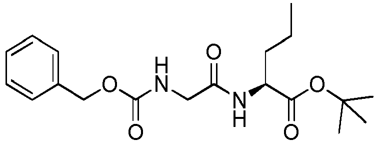

To a solution of L-norvaline (500 mg, 2.39 mmol) and 12 mL of THF at 25 °C was added 2,5-dioxopyrrolidin-1-yl-2-((benzyloxy)carbonyl)amino acetate (730 mg, 2.39mmol) and triethylamine (0.66 mL, 4.78 mmol). The resulting mixture was stirred at 25 °C for 2 hours. The mixture was quenched with water (5 mL) then extracted with ethyl acetate (30 mL). The layers were separated, and the organic layer was washed with water, brine, dried over Na₂SO₄, filtered, and concentrated under reduced pressure. The resulting product was purified by medium pressure liquid chromatography on silica gel using ethyl acetate : hexanes (1:1) to yield **Intermediate 1** as clear oil. ¹H NMR (CD₃OD, 600MHz) δ: 7.31-7.37 (m, 4H), 7.26-7.29 (m, 1H), 5.09 (s, 2H), 4.24-4.33 (m, 1H), 3.74-3.87 (m, 2H), 1.69-1.79 (m, 1H), 1.59-1.67 (m, 1H), 1.44 (s, 9H), 1.32-1.41 (m, 2H), 0.92 (t, J=7.3 Hz, 3H).

### Example B

### Intermediate 2

### (S)-tert-butyl 2-(2-aminoacetamido)pentanoate

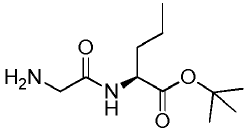

To a solution of **Intermediate 1** (760 mg, 2.09 mmol) and 35 mL of methanol at 25 °C was added 10% palladium on carbon (85mg) and a hydrogen balloon. The resulting mixture was stirred at 25 °C for 12 hours. The mixture was filtered through Celite® and concentrated under reduced pressure to yield Intermediate 2 as a clear oil. ¹H NMR (CD₃OD, 600MHz) δ: 4.23-4.34 (m, 1H), 3.29-3.30 (m, 2H), 1.70-1.80 (m, 1H), 1.59-1.68 (m, 1H), 1.45 (s, 9H), 1.35-1.43 (m, 2H), 0.91-0.97 (m, 3H).

### Example 1

### Compound 1

### (S)-2-(2-(3-(4-bromophenyl)ureido)acetamido)-4-methylpentanoic acid

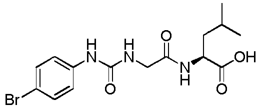

To a solution of glycyl-L-leucine (150 mg, 0.80 mmol) and 12 mL of methylene chloride at 25 °C was added 4-bromo-phenyl isocyanate (157 mg, 0.80mmol) and triethylamine (0.17 mL, 1.20 mmol). The resulting mixture was stirred at 25 °C for 12 hours. The mixture was concentrated and the residue was purified by medium pressure liquid chromatography on silica gel using methanol : dichloromethane (15:85) to yield **Compound 1** as white solid. ¹H NMR (CD₃OD, 300MHz) δ: 7.25-7.41 (m, 4H), 4.41-4.55 (m, 1H), 3.81-3.99 (m, 2H), 1.59-1.83 (m, 3H), 0.89-1.02 (m, 6H).

**Compounds 2, 3, 4** and **5** were prepared from the corresponding amino acid in a similar manner to the procedure described for **Example 1.** Intermediate (H-beta-Ala-Leu-OH), purchased from 3B Scientific, was used to prepare **Compound 5.**

### Example 2

### Compound 2

### (S)-4-methyl-2-(2-(3-(4-(trifluoromethyl)phenyl)ureido)acetamido)pentanoic acid

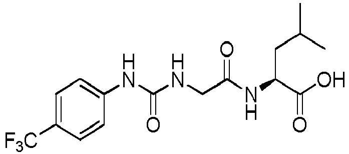

¹H NMR (CD₃OD, 600MHz) δ: 7.45-7.62 (m, 4H), 4.46 (dd, J=9.4, 5.3 Hz, 1H), 3.86-4.00 (m, 2H), 1.61-1.80 (m, 3H), 0.94 (dd, J=11.2, 6.5 Hz, 6H).

### Example 3

### Compound 3

### (S)-2-(2-(3-(4-bromophenyl)ureido)acetamido)-3-methylbutanoic acid

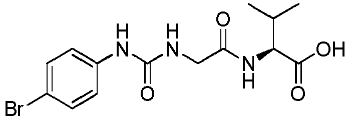

¹H NMR (CD₃OD, 600MHz) δ: 7.35-7.39 (m, 2H), 7.30-7.34 (m, 2H), 4.36 (d, J=5.3 Hz, 1H), 3.87-3.97 (m, 2H), 2.16-2.23 (m, 1H), 0.98 (d, J=7.0 Hz, 3H), 0.96 (d, J=7.0 Hz, 3H).

### Example 4

### Compound 4

### (S)-2-(2-(3-(4-bromophenyl)ureido)acetamido)-3-phenylpropanoic acid

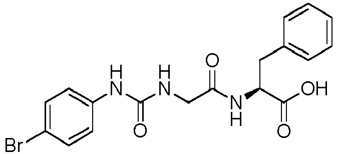

¹H NMR (CD₃OD, 600MHz) δ: 7.37 (d, J=8.8 Hz, 2H), 7.30-7.33 (m, 2H), 7.16-7.22 (m, 5H), 4.67 (dd, J=7.6, 5.3 Hz, 1H), 3.85-3.92 (m, 1H), 3.76-3.81 (m, 1H), 3.15-3.20 (m, 1H), 3.03 (dd, J=14.1, 7.6 Hz, 1H).

### Example 5

### Compound 5

### (S)-2-(3-(3-(4-bromophenyl)ureido)propanamido)-4-methylpentanoic acid

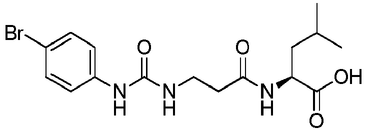

¹H NMR (CD₃OD, 600MHz) δ: 7.33-7.36 (m, 2H), 7.27-7.30 (m, 2H), 4.42 (t, J=6.7 Hz, 1H), 3.39-3.51 (m, 2H), 2.41-2.55 (m, 2H), 1.64-1.73 (m, 1H), 1.58-1.63 (m, 2H), 0.91 (dd, J=12.3, 6.5 Hz, 6H).

### Example 6

### Compound 6

### (S)-2-(2-(3-(4-bromophenyl)ureido)acetamido)pentanoic acid

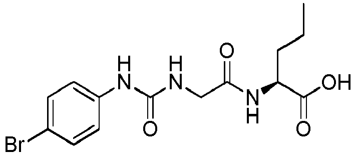

A solution of **Compound 8** (220 mg, 0.51 mmol) and 8mL of formic acid was stirred at 25 °C for 12 hours. The resulting reaction was quenched with water (10mL), and the product was extracted with EtOAc. The organic layer was washed with water, brine, dried over Na₂SO₄, filtered, and concentrated under reduced pressure. The residue was rinsed two times with acetone : hexane (2:98) to yield **Compound 6** as a white solid. ¹H NMR (CD₃OD, 600MHz) δ: 7.35 (s, 2H), 7.30 (d, J=9.4 Hz, 2H), 4.38-4.45 (m, 1H), 3.83-3.97 (m, 2H), 1.79-1.87 (m, 1H), 1.64-1.73 (m, 1H), 1.36-1.47 (m, 2H), 0.90-0.98 (m, 3H).

### Example 7

### Compound 7

### (S)-2-(2-(3-(4-(trifluoromethyl)phenyl)ureido)acetamido)pentanoic acid

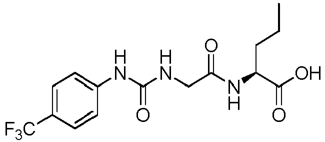

**Compound 7** was prepared from the corresponding *tert*-butyl ester in a similar manner to the procedure described for **Compound 6.** ¹H NMR (CD₃OD, 600MHz) δ: 7.54-7.57 (m, 2H), 7.50-7.53 (m, 2H), 4.37-4.44 (m, 1H), 3.88-3.98 (m, 2H), 1.82 (dddd, J=13.9, 9.3, 6.7, 4.7 Hz, 1H), 1.64-1.74 (m, 1H), 1.35-1.48 (m, 2H), 0.93 (t, J=7.3 Hz, 3H).

### Example 8

### Compound 8

### (S)-tert-butyl 2-(2-(3-(4-bromophenyl)ureido)acetamido)pentanoate

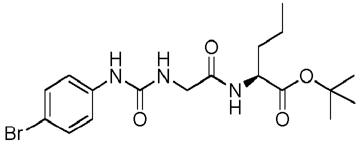

To a solution of **Intermediate 2** (182 mg, 0.79 mmol) and 7 mL of methylene chloride at 25 °C was added 4-bromo-phenyl isocyanate (156 mg, 0.79 mmol) and triethylamine (0.17 mL, 1.60 mmol). The resulting mixture was stirred at 25 °C for 12 hours. The mixture was concentrated and the residue was purified by medium pressure liquid chromatography on silica gel using ethyl acetate : hexane(1:1) to yield **Compound 8** as white solid. ¹H NMR (CD₃OD, 600MHz) δ: 7.35 (s, 2H), 7.29-7.32 (m, 2H), 4.24-4.33 (m, 1H), 3.89 (q, J=16.6 Hz, 2H), 1.72-1.81 (m, 1H), 1.60-1.69 (m, 1H), 1.44 (s, 9H), 1.34-1.41 (m, 2H), 0.93 (t, J=7.3 Hz, 3H).

### Example 9

### Compound 9

### (S)-tert-butyl 2-(2-(3-(4-(trifluoromethyl)phenyl)ureido)acetamido) pentanoate

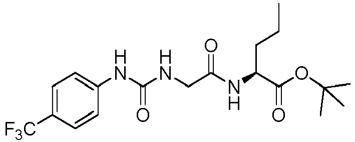

**Compound 9** was prepared from the corresponding amine in a similar manner to the procedure described for **Compound 8.** ¹H NMR (CD₃OD, 600MHz) δ: 7.54-7.57 (m, 2H), 7.50-7.52 (m, 2H), 4.26-4.32 (m, 1H), 3.86-3.98 (m, 2H), 1.73-1.80 (m, 1H), 1.61-1.69 (m, 1H), 1.45 (s, 9H), 1.37-1.43 (m, 2H), 0.93 (t, J=7.3 Hz, 3H).

### Biological Data

Biological activity of some specific compounds of the invention is set forth in **Table 1** below. CHO-Gα16 cells stably expressing FPR1 or FPR2 were cultured in (F12, 10% FBS, 1% PSA, 400 µg/ml geneticin and 50 µg/ml hygromycin). In general, the day before the experiment, 18,000 cells/well were plated in a 384-well clear bottom poly-D-lysine coated plate. The following day the screening compound-induced calcium activity was assayed on the FLIPR^{Tetra}. The drug plates were prepared in 384-well microplates using the EP3 and the MultiPROBE robotic liquid handling systems. Compounds were tested at concentrations ranging from 0.61 to 10,000 nM. Results are expressed as EC₅₀ (nM) and efficacy values.

**Table 1**

| **Structure** | **IUPAC Name Compound** | **FPR2 Ga16-HEK293 EC₅₀(nM) (Rel. eff.)** | **FPR1 Ga16-HEK293 EC₅₀(nM) (Rel. eff.)** |
|---|---|---|---|
| 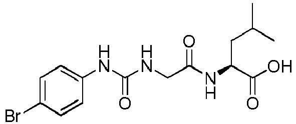 | (S)-2-(2-(3-(4-bromophenyl)ureido)acetami do)-4-methylpentanoic acid | **224 nM (0.91)** | **0.58 nM (1.00)** |
| 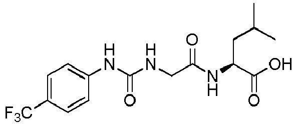 | (S)-4-methyl-2-(2-(3-(4-(trifluoromethyl)phenyl)ureid o)acetamido)pentanoic acid | **213 nM (0.96)** | **0.59 nM (0.96)** |
| 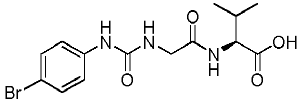 | (S)-2-(2-(3-(4-bromophenyl)ureido)acetami do)-3-methylbutanoic acid | **241 nM (0.96)** | **1 nM (1.04)** |
| 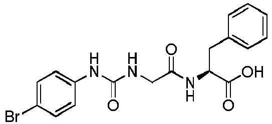 | (S)-2-(2-(3-(4-bromophenyl)ureido )acetami do)-3-phenylpropanoic acid | **356 nM (1.07)** | **14.7 nM (0.97)** |
| 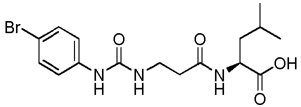 | (S)-2-(3-(3-(4-bromophenyl)ureido)propan amido)-4-methylpentanoic acid | **5629 nM (0.81)** | **76 nM (0.99)** |
| 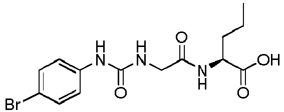 | (S)-2-(2-(3-(4-bromophenyl)ureido)acetami do)pentanoic acid | **308 nM (1.05)** | **4.93 nM (0.98)** |
| 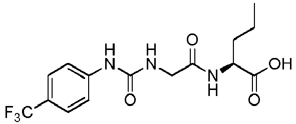 | (S)-2-(2-(3-(4-(trifluoromethyl)phenyl)ureid o)acetamido)pentanoic acid | **554 nM (1.01)** | **3.29 nM (0.90)** |
| 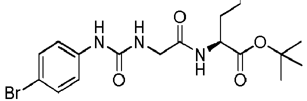 | (S)-tert-butyl 2-(2-(3-(4-bromophenyl)ureido)acetami do)pentanoate | **598 nM (0.97)** | **2303 nM (0.82)** |
| 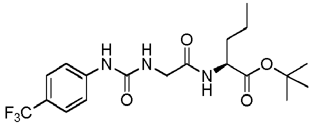 | (S)-tert-butyl 2-(2-(3-(4-(trifluoromethyl)phenyl)ureid o)acetamido) pentanoate | **1248 nM (1.00)** | **5145 nM (0.92)** |

## Claims

1. A compound of **Formula I:** 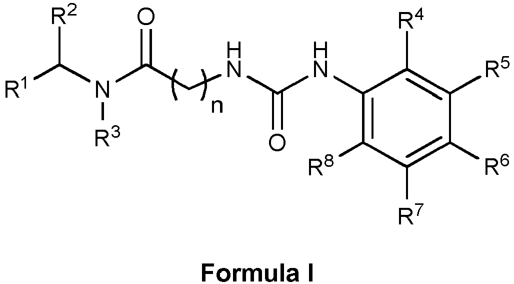 wherein:
R¹ is -COOH or -C(O)OR^{a}, wherein R^{a} is optionally substituted C₁₋₆ alkyl; wherein said optional alkyl substituent is selected from OH, halogen, -OC₁₋₈alkyl and -(O(CH₂)₁₋₈)_{q}-OC₁₋₈ alkyl; and q is 1, 2, 3, 4, 5 or 6;
R² is optionally substituted C₁₋₆ alkyl, wherein said optional alkyl substituent is selected from -OH, -SH, -OC₁₋₆ alkyl, -SC₁₋₆ alkyl, -COOH, -C(O)OC₁₋₆ alkyl, -C(O)NH₂, C₃₋₈cycloalkyl, C₃₋₈cycloalkenyl, C₆₋₁₀aryl, and heterocycle;
R³ is H or unsubstituted C₁₋₆ alkyl;
R⁴ is H, C₁₋₆ alkyl, C₁₋₆ haloalkyl, C₃₋₈ cycloalkyl, C₃₋₈ cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
R⁵ is H, C₁₋₆ alkyl, C₁₋₆ haloalkyl, halogen, -S(O)ₘR⁹ or -C(O)R¹⁰;
R⁶ is C₁₋₆ alkyl, C₁₋₆ haloalkyl, C₃₋₈ cycloalkyl, C₃₋₈ cycloalkenyl, C₆₋₁₀ aryl, heterocycle, halogen, -S(O)ₘR⁹, -C(O)R¹⁰ or -OR¹¹;
R⁷ is H, C₁₋₆ alkyl, C₁₋₆ haloalkyl, halogen, -S(O)ₘR⁹ or -C(O)R¹⁰;
R⁸ is H, C₁₋₆ alkyl, C₁₋₆ haloalkyl, C₃₋₈ cycloalkyl, C₃₋₈ cycloalkenyl, C₆₋₁₀ aryl, heterocycle, halogen, -NR¹¹R¹², -S(O)ₘR⁹, -C(O)R¹⁰, -C(O)OC₁₋₆ alkyl, -C(O)SC₁₋₆ alkyl, -OR¹³ or -SR¹³;
each R⁹ is independently -OH, C₁₋₆ alkyl or C₆₋₁₀ aryl;
each R¹⁰ is independently -OH, C₁₋₆ alkyl or C₆₋₁₀ aryl;
each R¹¹ is independently H, C₁₋₈ alkyl, C₃₋₈cycloalkyl, C₃₋₈cycloalkenyl, C₆₋₁₀ aryl or heterocycle;
each R¹² is independently H, C₁₋₈ alkyl, C₃₋₈cycloalkyl, C₃₋₈cycloalkenyl, C₆₋₁₀ aryl or heterocycle;
each R¹³ is independently H or C₁₋₈ alkyl;
each m is independently 1 or 2; and
n is 1, 2 or 3;
or a single enantiomer thereof;
or a mixture of enantiomers thereof;
or a tautomer of the foregoing;
or pharmaceutically acceptable salt of the foregoing;
provided that the compound is not:

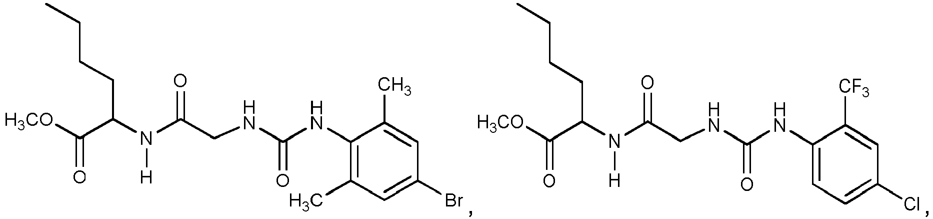

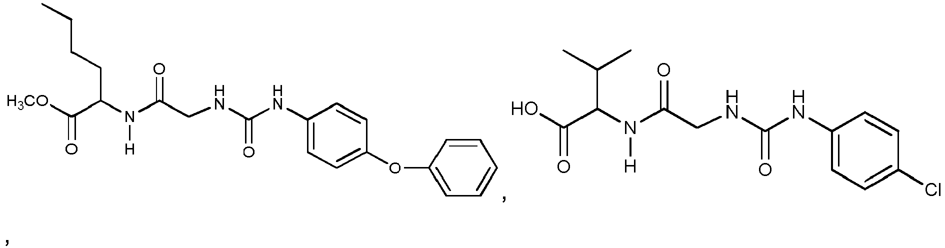

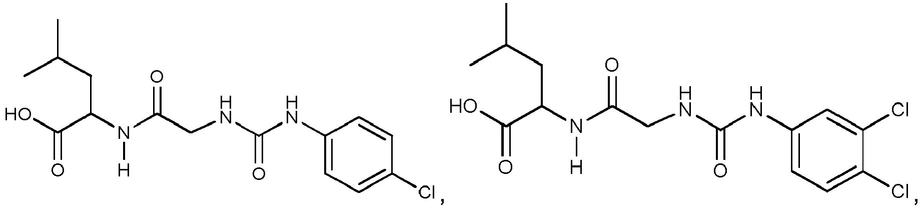

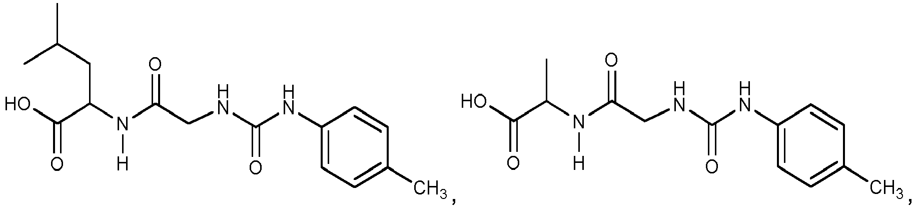

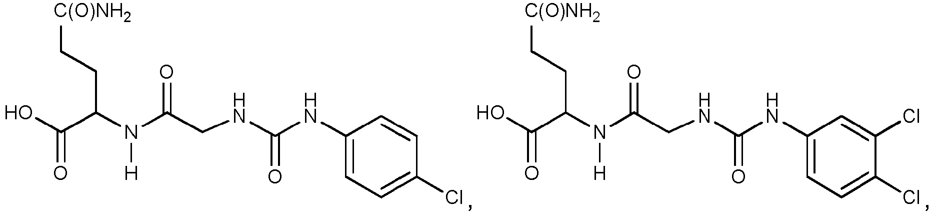

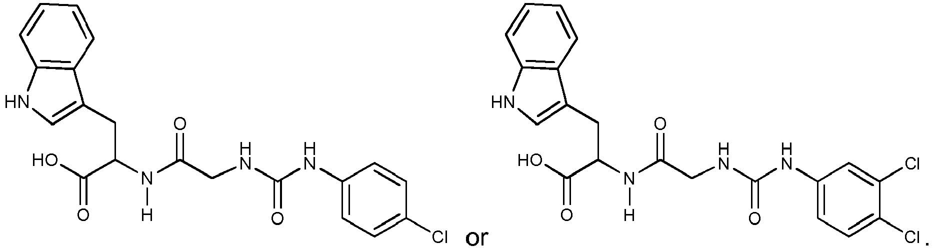

2. The compound of claim 1, wherein R¹ is -COOH.

3. The compound of any preceding claim, wherein R² is unsubstituted C₁₋₆ alkyl or benzyl and/or R³ is H or methyl.

4. The compound of any preceding claim, wherein n is 1 or 2.

5. The compound of any preceding claim, wherein R⁴ is H, F or C₁₋₆ haloalkyl; R⁵ is H, F or C₁₋₆ haloalkyl; R⁷ is H, F or C₁₋₆ haloalkyl; and R⁸ is H, F or C₁₋₆ haloalkyl.

6. The compound of claim 1 or 5, wherein each of R⁴, R⁵, R⁷ and R⁸ is H.

7. The compound of any preceding claim, wherein R⁶ is C₁₋₆ haloalkyl or halogen.

8. The compound of claim 1, provided that R⁶ is not chlorine, methyl or -OC₆₋₁₀ aryl.

9. The compound of claim 1, wherein:
R¹ is -COOH or -C(O)OR^{a}; wherein R^{a} is optionally substituted C₁₋₆ alkyl, wherein said optional alkyl substituent is selected from OH, halogen, -OC₁₋₈ alkyl and -(O(CH₂)₁₋₈)_{q}-OC₁₋₈ alkyl; and q is 1, 2, 3, 4, 5 or 6;
R² is unsubstituted C₁₋₆ alkyl or benzyl;
R³ is H or unsubstituted C₁₋₆ alkyl;
R⁴ is H or halogen;
R⁵ is H;
R⁶ is C₁₋₆ haloalkyl or bromine;
R⁷ is H;
R⁸ is H or halogen; and
n is 1 or 2;
or a single enantiomer thereof;
or a mixture of enantiomers thereof;
or a tautomer of the foregoing;
or pharmaceutically acceptable salt of the foregoing.

10. The compound of claim 9, wherein R¹ is -COOH.

11. The compound of claim 9 or 10, wherein R⁴ and R⁸ is H.

12. The compound of claim 9, wherein:
R¹ is -COOH or -C(O)OR^{a}, wherein R^{a} is unsubstituted C₁₋₆ alkyl;
R² is unsubstituted C₁₋₃ alkyl or benzyl;
R³ is H or -CH₃;
R⁴ is H;
R⁵ is H;
R⁶ is -CF₃ or bromine;
R⁷ is H;
R⁸ is H; and
n is 1 or 2;
or a tautomer thereof;
or pharmaceutically acceptable salt of any of the foregoing.

13. The compound of claim 1, selected from: 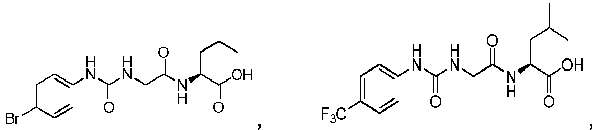 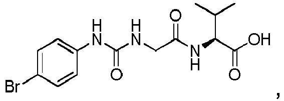 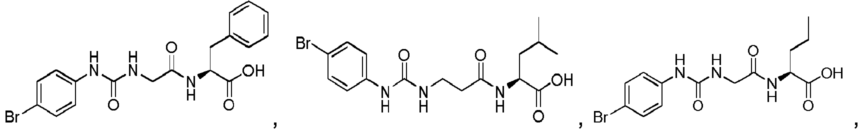 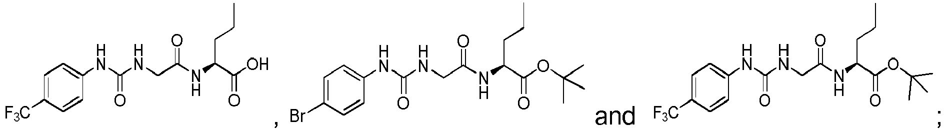 and tautomers thereof;
and pharmaceutically acceptable salts thereof.

14. A pharmaceutical composition comprising a compound of any one of claims 1 through 13 and a pharmaceutically acceptable excipient.

15. A compound or composition of any one of claims 1 through 14 for use in a method of treating a disease or condition alleviated by FPR modulation, the disease or condition is preferably an ocular disease or condition selected from: ocular inflammation, age-related macular degeneration, wet macular degeneration, dry macular degeneration, uveitis, dry eye, keratitis, allergic eye disease and conditions affecting the posterior part of the eye, maculopathies and retinal degeneration, non-exudative age related macular degeneration, exudative age related macular degeneration, choroidal neovascularization, diabetic retinopathy (proliferative), retinopathy of prematurity, acute macular neuroretinopathy, central serous chorioretinopathy, cystoid macular edema, diabetic macular edema, infectious keratitis, herpetic keratitis, corneal angiogenesis, lymphangiogenesis, retinitis, choroiditis, acute multifocal placoid pigment epitheliopathy, Behcet's disease, birdshot retinochoroidopathy, infectious (syphilis, lyme, tuberculosis, toxoplasmosis), intermediate uveitis (pars planitis), multifocal choroiditis, multiple evanescent white dot syndrome (mewds), ocular sarcoidosis, posterior scleritis, serpiginous choroiditis, subretinal fibrosis and uveitis syndrome, Vogt-Koyanagi-and Harada syndrome; vascular diseases/ exudative diseases such as retinal arterial occlusive disease, central retinal vein occlusion, cystoids macular edema, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angiitis, sickle cell retinopathy and other hemoglobinopathies, angioid streaks, familial exudative vitreoretinopathy, Eales disease, traumatic/surgical conditions of the eye, sympathetic ophthalmia, uveitic retinal disease, retinal detachment, trauma, conditions caused by laser, conditions caused by photodynamic therapy, photocoagulation, hypoperfusion during surgery, radiation retinopathy, bone marrow transplant retinopathy, post-surgical corneal wound healing or inflammation, post-cataract surgical inflammation, proliferative disorders of the eye, proliferative vitreal retinopathy and epiretinal membranes, proliferative diabetic retinopathy, infectious disorders of the eye, ocular histoplasmosis, ocular toxocariasis, presumed ocular histoplasmosis syndrome (POHS), endophthalmitis, toxoplasmosis, retinal diseases associated with HIV infection, choroidal disease associate with HIV infection, uveitic disease associate with HIV infection, viral retinitis, acute retinal necrosis, progressive outer retinal necrosis, fungal retinal diseases, ocular syphilis, ocular tuberculosis, diffuse unilateral subacute neuroretinitis, myiasis, genetic disorders affecting the eye, retinitis pigmentosa, systemic disorders with associated retinal dystrophies, congenital stationary night blindness, cone dystrophies, Stargardt's disease, fundus flavimaculatus, Best's disease, pattern dystrophy of the retinal pigmented epithelium, X-linked retinoschisis, Sorsby's fundus dystrophy, benign concentric maculopathy, Bietti's crystalline dystrophy, pseudoxanthoma elasticum, retinal tears, retinal holes, retinal detachment, macular hole, giant retinal tear, ocular tumors, retinal disease associated with tumors, congenital hypertrophy of the retinal pigmented epithelium, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, intraocular lymphoid tumors, miscellaneous other diseases affecting the posterior part of the eye, punctate inner choroidopathy, acute posterior multifocal placoid pigment epitheliopathy, myopic retinal degeneration, acute retinal pigment epitheliitis, post-surgical corneal inflammation, blepharitis, meibomian gland dysfunction (MGD), corneal wound healing, , glaucoma, branch vein occlusion, Best's vitelliform macular degeneration, retinitis pigmentosa, proliferative vitreoretinopathy (PVR), and any other degenerative disease of either the photoreceptors or the retinal pigment epithelium, a dermal disease or condition selected from dermal inflammation, a dermal wound, hypertrophic scars, keloids, burns, sunburn, rosacea, erythema of the skin, atopic dermatitis, acne, psoriasis, seborrheic dermatitis, actinic keratoses, basal cell carcinoma, squamous cell carcinoma, melanoma, viral warts, photoaging, photodamage, melasma, post-inflammatory hyperpigmentation, disorders of pigmentation, and alopecia, scarring and non-scarring forms or the disease or condition is selected from a systemic inflammatory disease, stroke, coronary artery disease, a cardiovascular disorder, angina pectoris, an obstructive airway disease, a neurological disorder, a central nervous system disorder, Alzheimer's disease, neuroinflammation, pain, an HIV-mediated retroviral infection, an immunological disorder, arthritis, rheumatoid arthritis, systemic lupus erythematosus, multiple sclerosis, sepsis, inflammatory bowel disease, ulcerative colitis, asthma, an allergic disorder and cachexia.

## Patentansprüche

1. Verbindung der Formel I: 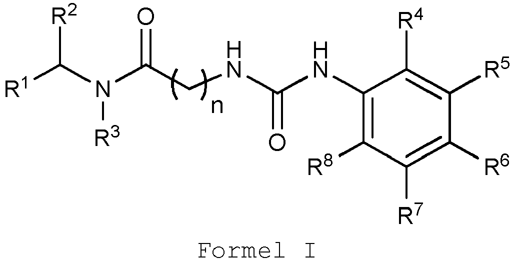 worin:
R¹ für-COOH oder -C(O)OR^{a} steht, worin R^{a} für optional substituiertes C₁₋₆-Alkyl steht; worin der optionale Substituent des Alkyls ausgewählt ist aus OH, Halogen, -OC₁₋₈-Alkyl und - (O (CH₂)₁₋₈)_{q}-OC₁₋₈-Alkyl; und q für 1, 2, 3, 4, 5 oder 6 steht;
R² für optional substituiertes C₁₋₆-Alkyl steht, worin der optionale Substituent des Alkyls ausgewählt ist aus -OH, -SH, -OC₁₋₆-Alkyl, -SC₁₋₆-Alkyl, -COOH, - C(O) OC₁₋₆-Alkyl, -C(O)NH₂, C₃₋₈-Cycloalkyl, C₃₋₈-Cycloalkenyl, C₆₋₁₀-Aryl und Heterocyclus;
R³ für H oder unsubstituiertes C₁₋₆-Alkyl steht;
R⁴ für H, C₁₋₆-Alkyl, C₁₋₆-Halogenalkyl, C₃₋₈-Cycloalkyl, C₃₋₈-Cycloalkenyl, C₆₋₁₀-Aryl, Heterocyclus, Halogen, -NR¹¹R¹², -S(O)ₘR⁹, -C(O)R¹⁰), -C(O)OC₁₋₆-Alkyl, -C(O)SC₁₋₆-AAlkyl, -OR¹³ oder -SR¹³ steht;
R⁵ für H, C₁₋₆-Alkyl, C₁₋₆-Halogenalkyl, Halogen, -S(O)ₘR⁹ oder -C(O)R¹⁰ steht;
R⁶ für C₁₋₆-Alkyl, C₁₋₆-Halogenalkyl, C₃₋₈-Cycloalkyl, C₃₋₈-Cycloalkenyl, C₆₋₁₀-Aryl, Heterocyclus, Halogen, -S(O)ₘR⁹, -C(O)R¹⁰ oder -OR¹¹ steht;
R⁷ für H, C₁₋₆-Alkyl, C₁₋₆-Halogenalkyl, Halogen, -S(O)ₘR⁹ oder -C(O)R¹⁰ steht;
R⁸ für H, C₁₋₆-Alkyl, C₁₋₆-Halogenalkyl, C₃₋₈-Cycloalkyl, C₃₋₈-Cycloalkenyl, C₆₋₁₀-Aryl, Heterocyclus, Halogen, -NR¹¹R¹², -S(O)ₘR⁹, -C(O)R¹⁰), -C(O)OC₁₋₆-Alkyl, -C(O)SC₁₋₆-Alkyl, -OR¹³ oder -SR¹³ steht;
jedes R⁹ unabhängig für -OH, C₁₋₆-Alkyl oder C₆₋₁₀-Aryl steht;
jedes R¹⁰ unabhängig für -OH, C₁₋₆-Alkyl oder C₆₋₁₀-Aryl steht;
jedes R¹¹ unabhängig für H, C₁₋₈-Alkyl, C₃₋₈-Cycloalkyl, C₃₋₈-Cycloalkenyl, C₆₋₁₀-Aryl oder Heterocyclus steht;
jedes R¹² unabhängig für H, C₁₋₈-Alkyl, C₃₋₈-Cycloalkyl, C₃₋₈-Cycloalkenyl, C₆₋₁₀-Aryl oder Heterocyclus steht;
jedes R¹³ unabhängig für H oder C₁₋₈-Alkyl steht;
jedes m unabhängig für 1 oder 2 steht; und
n für 1, 2 oder 3 steht;
oder ein einzelnes Enantiomer davon;
oder ein Gemisch an Enantiomeren davon;
oder ein Tautomer des zuvor Genannten;
oder ein pharmazeutisch annehmbares Salz des zuvor Genannten;
mit der Maßgabe, dass die Verbindung nicht: 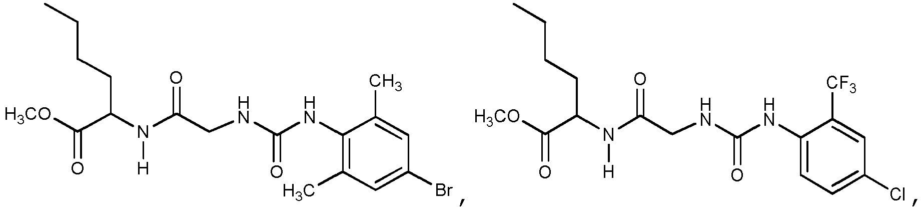 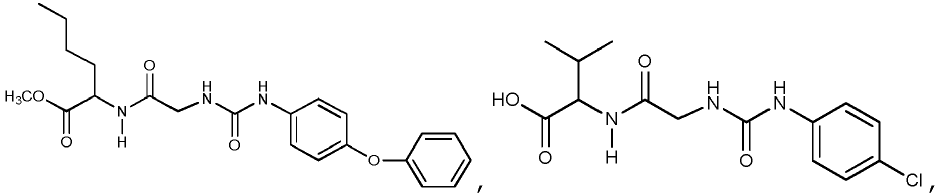 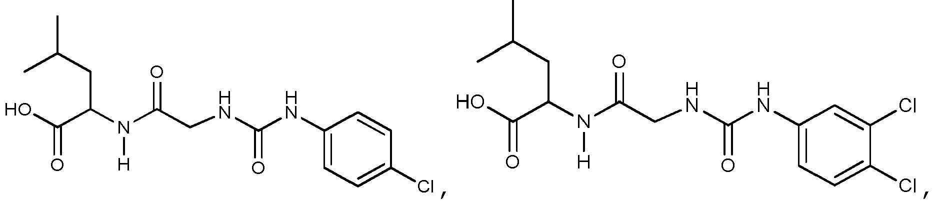 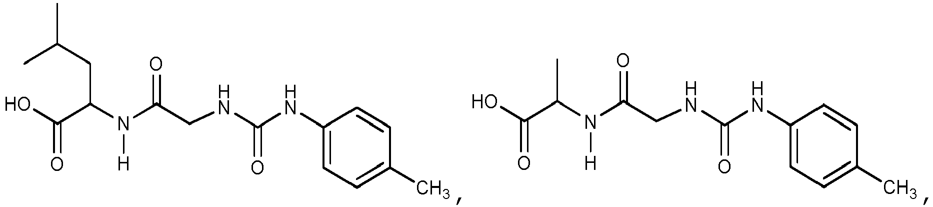 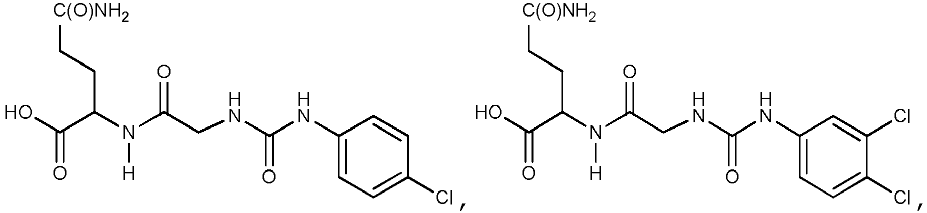 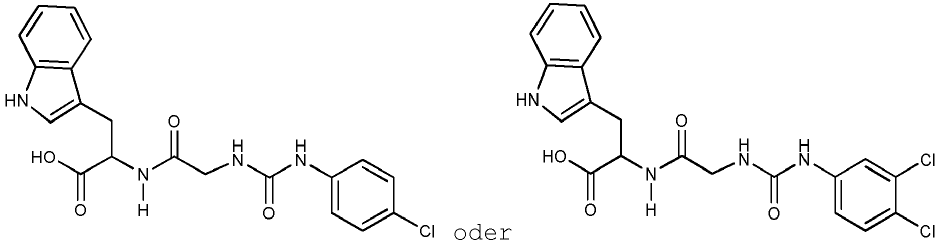
ist.

2. Verbindung gemäß Anspruch 1, worin R¹ für -COOH steht.

3. Verbindung gemäß einem der vorhergehenden Ansprüche, worin R² für unsubstituiertes C₁₋₆-Alkyl oder Benzyl steht und/oder R³ für H oder Methyl steht.

4. Verbindung gemäß einem der vorhergehenden Ansprüche, worin n für 1 oder 2 steht.

5. Verbindung gemäß einem der vorhergehenden Ansprüche, worin R⁴ für H, F oder C₁₋₆-Halogenalkyl steht; R⁵ für H, F oder C₁₋₆-Halogenalkyl steht; R⁷ für H, F oder C₁₋₆-Halogenalkyl steht; und R⁸ für H, F oder C₁₋₆-Halogenalkyl steht.

6. Verbindung gemäß Anspruch 1 oder 5, worin jedes aus R⁴, R⁵, R⁷ und R⁸ für H steht.

7. Verbindung gemäß einem der vorhergehenden Ansprüche, worin R⁶ für C₁₋₆-Halogenalkyl oder Halogen steht.

8. Verbindung gemäß Anspruch 1, mit der Maßgabe, dass R⁶ nicht für Chlor, Methyl oder -OC₆₋₁₀-Aryl steht.

9. Verbindung gemäß Anspruch 1, worin:
R¹ für -COOH oder -C(O)OR^{a} steht; worin R^{a} für optional substituiertes C₁₋₆-Alkyl steht, worin der optionale Substituent des Alkyls ausgewählt ist aus OH, Halogen, -OC₁₋₈-Alkyl und - (O(CH₂)₁₋₈)_{q}-OC₁₋₈-Alkyl; und q für 1, 2, 3, 4, 5 oder 6 steht;
R² für unsubstituiertes C₁₋₆-Alkyl oder Benzyl steht;
R³ für H oder unsubstituiertes C₁₋₆-Alkyl steht;
R⁴ für H oder Halogen steht;
R⁵ für H steht;
R⁶ für C₁₋₆-Halogenalkyl oder Brom steht;
R⁷ für H steht;
R⁸ für H oder Halogen steht; und
n für 1 oder 2 steht;
oder ein einzelnes Enantiomer davon;
oder ein Gemisch an Enantiomeren davon;
oder ein Tautomer des zuvor Genannten;
oder ein pharmazeutisch annehmbares Salz des zuvor Genannten.

10. Verbindung gemäß Anspruch 9, worin R¹ für -COOH steht.

11. Verbindung gemäß Anspruch 9 oder 10, worin R⁴ und R⁸ für H stehen.

12. Verbindung gemäß Anspruch 9, worin:
R¹ für -COOH oder -C(O)OR^{a} steht, worin R^{a} für unsubstituiertes C₁₋₆-Alkyl steht;
R² für unsubstituiertes C₁₋₃-Alkyl oder Benzyl steht;
R³ für H oder -CH₃ steht;
R⁴ für H steht;
R⁵ für H steht;
R⁶ für -CF₃ oder Brom steht;
R⁷ für H steht;
R⁸ für H steht; und
n für 1 oder 2 steht;
oder ein Tautomer davon;
oder ein pharmazeutisch annehmbares Salz des zuvor Genannten.

13. Verbindung gemäß Anspruch 1, die ausgewählt ist aus: 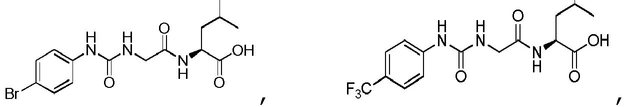 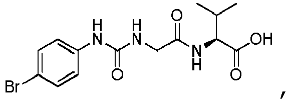 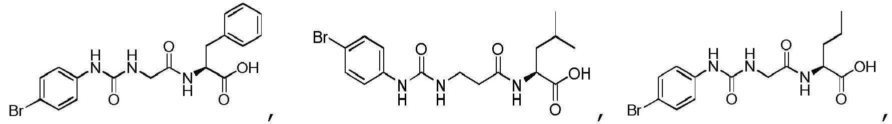 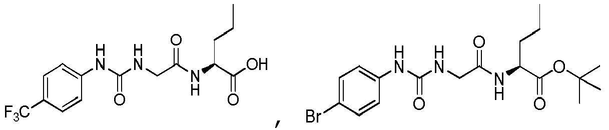 und 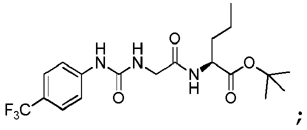 und Tautomeren davon;
und pharmazeutisch annehmbaren Salzen davon.

14. Pharmazeutische Zusammensetzung, umfassend eine Verbindung gemäß einem der Ansprüche 1 bis 13 und einen pharmazeutisch annehmbaren Hilfsstoff.

15. Verbindung oder Zusammensetzung gemäß einem der Ansprüche 1 bis 14 zur Verwendung in einem Verfahren zur Behandlung einer Erkrankung oder eines Zustands, der/die durch FPR-Modulation gelindert wird, wobei die Krankheit oder der Zustand vorzugsweise eine Augenkrankheit oder ein das Auge betreffender Zustand ist, ausgewählt aus:
Augenentzündung, altersbedingter Makuladegeneration, feuchter Makuladegeneration, trockener Makuladegeneration, Uveitis, trockenem Auge, Keratitis, allergischer Augenkrankheit und Erkrankungen des hinteren Teils des Auges, Makulopathien und Netzhautdegeneration, nicht exsudativer altersbedingter Makuladegeneration, exsudativer altersbedingter Makuladegeneration, choroidaler Neovaskularisation, diabetischer Retinopathie (proliferativ), Frühgeborenen-Retinopathie, akuter makulärer Neuroretinopathie, Chorioretinopathia centralis serosa, zystoidem Makulaödem, diabetischem Makulaödem, infektiöser Keratitis, herpetischer Keratitis, Hornhautangiogenese, Lymphangiogenese, Retinitis, Choroiditis, akuter multifokaler plakoider Pigmentepitheliopathie, Behcet-Krankheit, Birdshot-Retinochoroidopathie, Infektionen (Syphilis, Lyme, Tuberkulose, Toxoplasmose), intermediärer Uveitis (Pars Planitis), multifokaler Choroiditis, multiplem Syndrom der flüchtigen weißen Flecken (Mewds), Augensarkoidose, posteriorer Skleritis, serpiginöser Choroiditis, subretinalem Fibrose- und Uveitis-Syndrom, Vogt-Koyanagi- und Harada-Syndrom; Gefäßerkrankungen/exsudativen Erkrankungen wie Verschlusskrankheit der Netzhautarterien, Verschluss der zentralen Netzhautvene, zystoidem Makulaödem, disseminierter intravaskulärer Koagulopathie, Netzhautastvenenverschluss, hypertensiven Fundusveränderungen, ischämischem Augensyndrom, arteriellen Mikroaneurysmen des Auges, Morbus Coat, parafovealer Teleangiektasie, hemi-retinalem Venenverschluss, Papillophlebitis, Verschluss der zentralen Netzhautarterie, Netzhautastarterienverschluss, Karotisarterienerkrankung (CAD), Frosted-Branch-Angiitis, Sichelzellenretinopathie und anderen Hämoglobinopathien, Angioid-Streaks, familiärer exsudativer Vitreoretinopathie, Eales-Krankheit, traumatischen/chirurgischen Zuständen des Auges, sympathischer Ophthalmie, uveitischer Netzhauterkrankung, Netzhautablösung, Trauma, durch Laser verursachten Zuständen, durch photodynamische Therapie verursachten Zuständen, Photokoagulation, Hypoperfusion während der Operation, Strahlenretinopathie, Knochenmarktransplantat-Retinopathie, postoperativer Hornhautwundheilung oder -entzündung, Entzündung nach chirurgischem Katarakteingriff, proliferativen Erkrankungen des Auges, proliferativer vitrealer Retinopathie und epiretinalen Membranen, proliferativer diabetischer Retinopathie, infektiösen Erkrankungen des Auges, Augenhistoplasmose, Augentoxokariose, vermutetem Augenhistoplasmosesyndrom (POHS), Endophthalmitis, Toxoplasmose, mit einer HIV-Infektion assoziierten Netzhauterkrankungen, mit einer HIV-Infektion assoziierter Aderhauterkrankung, mit einer HIV-Infektion assoziierter uveitischer Erkrankung, viraler Retinitis, akuter Netzhautnekrose, progressiver äußerer Netzhautnekrose, mykotischen Netzhauterkrankungen, Augensyphilis, Augentuberkulose, diffuser einseitiger subakuter Neuroretinitis, Myiasis, genetischen Störungen des Auges, Retinitis pigmentosa, systemischen Störungen mit assoziierten Netzhautdystrophien, angeborener stationärer Nachtblindheit, Zapfendystrophien, Stargardt-Krankheit, Fundus flavimaculatus, Best-Krankheit, Musterdystrophie des retinalen pigmentierten Epithels, X-chromosomaler Retinoschisis, Sorsby-Fundusdystrophie, gutartiger konzentrischer Makulopathie, Bietti-Kristalldystrophie, Pseudoxanthoma elasticum, Netzhautrissen, Netzhautlöchern, Netzhautablösung, Makulaloch, riesigem Netzhautriss, Augentumoren, mit Tumoren assoziierter Netzhauterkrankung, angeborener Hypertrophie des retinalen pigmentierten Epithels, posteriorem Uvealmelanom, Aderhauthämangiom, Aderhautosteom, Aderhautmetastasen, kombiniertem Hamartom der Netzhaut und retinalem pigmentiertem Epithel, Retinoblastom, vasoproliferativen Tumoren des Augenhintergrunds, Netzhautastrozytom, intraokularen lymphoiden Tumoren, verschiedenen anderen Erkrankungen, die den hinteren Teil des Auges betreffen, punktierter innerer Aderhautopathie, akuter posteriorer multifokaler plakoider Pigmentepitheliopathie, myoper Netzhautdegeneration, akuter Netzhautpigmentepitheliitis, postoperativer Hornhautentzündung, Blepharitis, Meibom-Drüsen-Dysfunktion (MGD), Hornhautwundheilung, Glaukom, Astvenenverschluss, Best-Vitelliform-Makuladegeneration, Retinitis pigmentosa, proliferativer Vitreoretinopathie (PVR) und jeder anderen degenerativer Erkrankung der Photorezeptoren oder des retinalen Pigmentepithels, eine Hautkrankheit oder ein Zustand, ausgewählt aus Hautentzündung, Hautwunde, hypertrophen Narben, Keloiden, Verbrennungen, Sonnenbrand, Rosacea, Hautrötung, atopischer Dermatitis, Akne, Psoriasis, seborrhoischer Dermatitis, aktinischer Keratosen, Basalzellkarzinom, Plattenepithelkarzinom, Melanom, Viruswarzen, Lichtalterung, Lichtschäden, Melasma, postinflammatorischer Hyperpigmentierung, Pigmentstörungen und Alopezie, narbenbildender und nichtnarbenbildender Formen oder die Krankheit oder der Zustand ausgewählt ist aus einer systemischen entzündlichen Erkrankung, Schlaganfall, einer Erkrankung der Herzkranzgefäße, einer Herz-Kreislauf-Störung, Angina pectoris, einer obstruktiven Atemwegserkrankung, einer neurologischen Störung, einer Störung des Zentralnervensystems, Alzheimer-Krankheit, Neuroinflammation, Schmerz, einer HIV-vermittelten retroviralen Infektion, einer immunologischen Störung, Arthritis, rheumatoider Arthritis, systemischem Lupus erythematodes, Multipler Sklerose, Sepsis, entzündlicher Darmerkrankung, Colitis ulcerosa, Asthma, einer allergischen Störung und Kachexie.

## Revendications

1. Composé de la formule I : 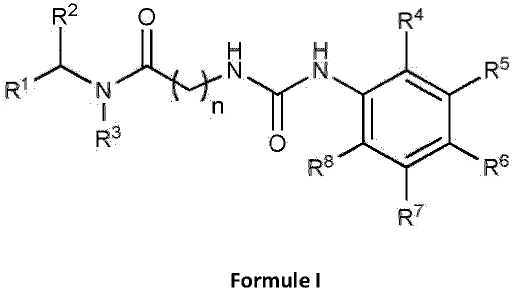 dans lequel :
R¹ est -COOH ou -C(O)OR^{a}, dans lequel R^{a} est alkyle en C₁₋₆ facultativement substitué ; dans lequel ledit substituant alkyle facultatif est choisi parmi OH, halogène, - Oalkyle en C₁₋₈ et -(O(CH₂)₁₋₈)_{q}-Oalkyle en C₁₋₈ ; et q vaut 1, 2, 3, 4, 5 ou 6 ;
R² est alkyle en C₁₋₆ facultativement substitué, dans lequel ledit substituant alkyle facultatif est choisi parmi -OH, -SH, -Oalkyle en C₁₋₆, -Salkyle en C₁₋₆, -COOH, -C(O)Oalkyle en C₁₋₆, -C(O)NH₂, cycloalkyle en C₃₋₈, cycloalcényle en C₃₋₈, aryle en C₆₋₁₀ et hétérocycle ;
R³ est H ou alkyle en C₁₋₆ non substitué ;
R⁴ est H, alkyle en C₁₋₆, halogénoalkyle en C₁₋₆, cycloalkyle en C₃₋₈, cycloalcényle en C₃₋₈, aryle en C₆₋₁₀, hétérocycle, halogène, -NR¹¹R¹², -S(O)ₘR⁹, -C(O)R¹⁰, -C(O)Oalkyle en C₁₋₆, -C(O)Salkyle en C₁₋₆, -OR¹³ ou -SR¹³ ;
R⁵ est H, alkyle en C₁₋₆, halogénoalkyle en C₁₋₆, halogène, -S(O)ₘR⁹ ou -C(O)R¹⁰ ;
R⁶ est alkyle en C₁₋₆, halogénoalkyle en C₁₋₆, cycloalkyle en C₃₋₈, cycloalcényle en C₃₋₈, aryle en C₆₋₁₀, hétérocycle, halogène, -S(O)ₘR⁹, -C(O)R¹⁰ ou -OR¹¹ ;
R⁷ est H, alkyle en C₁₋₆, halogénoalkyle en C₁₋₆, halogène, -S(O)ₘR⁹ ou -C(O)R¹⁰ ;
R⁸ est H, alkyle en C₁₋₆, halogénoalkyle en C₁₋₆, cycloalkyle en C₃₋₈, cycloalcényle en C₃₋₈, aryle en C₆₋₁₀, hétérocycle, halogène, -NR¹¹R¹², -S(O)ₘR⁹, -C(O)R¹⁰, -C(O)Oalkyle en C₁₋₆, -C(O)Salkyle en C₁₋₆, -OR¹³ ou -SR¹³ ;
chaque R⁹ est indépendamment -OH, alkyle en C₁₋₆ ou aryle en C₆₋₁₀ ;
chaque R¹⁰ est indépendamment -OH, alkyle en C₁₋₆ ou aryle en C₆₋₁₀ ;
chaque R¹¹ est indépendamment H, alkyle en C₁₋₈, cycloalkyle en C₃₋₈, cycloalcényle en C₃₋₈, aryle en C₆₋₁₀ ou hétérocycle ;
chaque R¹² est indépendamment H, alkyle en C₁₋₈, cycloalkyle en C₃₋₈, cycloalcényle en C₃₋₈, aryle en C₆₋₁₀ ou hétérocycle ;
chaque R¹³ est indépendamment H ou alkyle en C₁₋₈ ;
chaque m vaut indépendamment 1 ou 2 ; et
n vaut 1, 2 ou 3 ;
ou un énantiomère unique de celui-ci ;
ou un mélange d'énantiomères de celui-ci ;
ou un tautomère des précédents ;
ou un sel pharmaceutiquement acceptable des précédents ;
à condition que le composé ne soit pas :

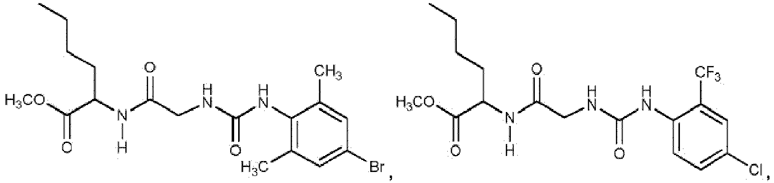

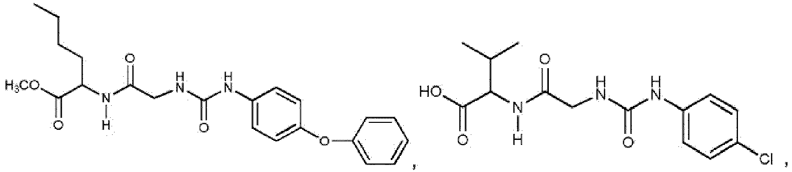

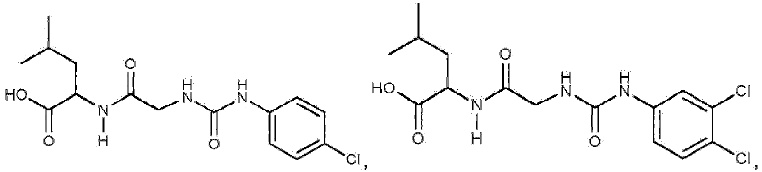

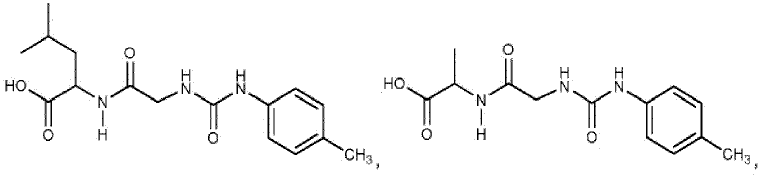

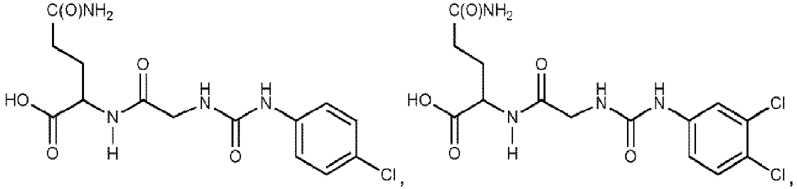

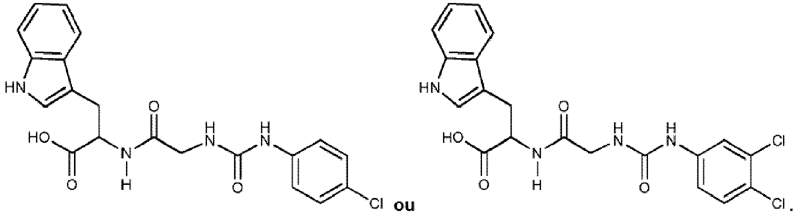

2. Composé selon la revendication 1, dans lequel R¹ est -COOH.

3. Composé selon une quelconque revendication précédente, dans lequel R² est alkyle en C₁₋₆ non substitué ou benzyle et/ ou R³ est H ou méthyle.

4. Composé selon une quelconque revendication précédente, dans lequel n vaut 1 ou 2.

5. Composé selon une quelconque revendication précédente, dans lequel R⁴ est H, F ou halogénoalkyle en C₁₋₆; R⁵ est H, F ou halogénoalkyle en C₁₋₆; R⁷ est H, F ou halogénoalkyle en C₁₋₆ ; et R⁸ est H, F ou halogénoalkyle en C₁₋₆.

6. Composé selon la revendication 1 ou 5, dans lequel chacun de R⁴, R⁵, R⁷ et R⁸ est H.

7. Composé selon une quelconque revendication précédente, dans lequel R⁶ est halogénoalkyle en C₁₋₆ ou halogène.

8. Composé selon la revendication 1, à condition que R⁶ ne soit pas chlore, méthyle ou -Oaryle en C₆₋₁₀.

9. Composé selon la revendication 1, dans lequel :
R¹ est -COOH ou -C(O)OR^{a} ; dans lequel R^{a} est alkyle en C₁₋₆ facultativement substitué,
dans lequel ledit substituant alkyle facultatif est choisi parmi OH, halogène, -Oalkyle en C₁₋₈ et -(O(CH₂)₁₋₈)_{q}-Oalkyle en C₁₋₈ ; et q vaut 1, 2, 3, 4, 5 ou 6 ;
R² est alkyle en C₁₋₆ non substitué ou benzyle ;
R³ est H ou alkyle en C₁₋₆ non substitué ;
R⁴ est H ou halogène ;
R⁵ est H ;
R⁶ est halogénoalkyle en C₁₋₆ ou brome ;
R⁷ est H ;
R⁸ est H ou halogène ; et
n vaut 1 ou 2 ;
ou un énantiomère unique de celui-ci ;
ou un mélange d'énantiomères de celui-ci ;
ou un tautomère des précédents ;
ou un sel pharmaceutiquement acceptable des précédents.

10. Composé selon la revendication 9, dans lequel R¹ est -COOH.

11. Composé selon la revendication 9 ou 10, dans lequel R⁴ et R⁸ sont H.

12. Composé selon la revendication 9, dans lequel :
R¹ est -COOH ou -C(O)OR^{a}, dans lequel R^{a} est alkyle en C₁₋₆ non substitué ;
R² est alkyle en C₁₋₃ non substitué ou benzyle ;
R³ est H ou -CH₃ ;
R⁴ est H ;
R⁵ est H ;
R⁶ est -CF₃ ou brome ;
R⁷ est H ;
R⁸ est H ; et
n est 1 ou 2 ;
ou un tautomère de celui-ci ;
ou un sel pharmaceutiquement acceptable de l'un quelconque des précédents.

13. Composé selon la revendication 1, choisi parmi : 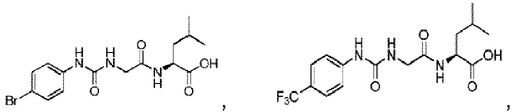 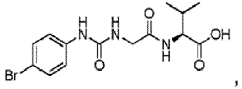 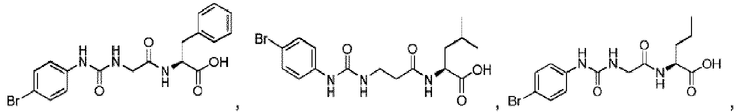 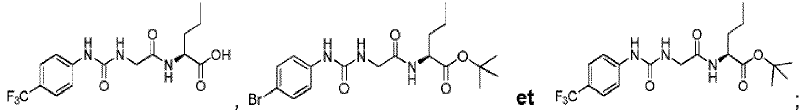 et leurs tautomères ;
et leurs sels pharmaceutiquement acceptables.

14. Composition pharmaceutique comprenant un composé de l'une quelconque des revendications 1 à 13, et un excipient pharmaceutiquement acceptable.

15. Composé ou composition selon l'une quelconque des revendications 1 à 14 pour une utilisation dans un procédé de traitement d'une maladie ou d'une affection soulagée par modulation de FPR, la maladie ou affection est de préférence une maladie ou affection oculaire choisie parmi : inflammation oculaire, dégénérescence maculaire liée à l'âge, dégénérescence maculaire humide, dégénérescence maculaire sèche, uvéite, syndrome de l'œil sec, kératite, maladie et affections allergiques de l'œil affectant la partie postérieure de l'œil, maculopathies et dégénérescence rétinienne, dégénérescence maculaire liée à l'âge non exsudative, dégénérescence maculaire liée à l'âge exsudative, néovascularisation choroïdienne, rétinopathie diabétique (proliférative), rétinopathie du prématuré, neurorétinopathie maculaire aiguë, choriorétinopathie séreuse centrale, œdème maculaire cystoïde, œdème maculaire diabétique, kératite infectieuse, kératite herpétique, angiogenèse cornéenne, lymphangiogenèse, rétinite, choroïdite, épithéliopathie pigmentaire placoïde multifocale aiguë, maladie de Behçet, rétinochoroïdopathie de type Birdshot, infections (syphilis, Lyme, turberculose, toxoplasmose), uvéite intermédiaire (pars planitis), choroïdite multifocale, syndrome des points blancs évanescents multiples (MEWDS), sarcoïdose oculaire, sclérite postérieure, choroïdite serpigineuse, fibrose subrétinienne et syndrome de l'uvéite, syndrome de Vogt-Koyanagi-Harada ; maladies vasculaires/maladies exsudatives telles que la maladie occlusive artérielle rétinienne, occlusion de la veine rétinienne centrale, œdème maculaire des cystoïdes, coagulopathie intravasculaire disséminée, occlusion de la veine rétinienne brachiale, variations d'hypertension du fond d'œil, syndrome ischémique oculaire, microanévrismes des artères rétiniennes, maladie de Coat, télangiectasie parafovéale, occlusion de la veine hémi-rétinienne, papillophlébite, occlusion de l'artère rétinienne centrale, occlusion de l'artère rétinienne branchiale, maladie carotidienne (CAD), angéite givrée, rétinopathie drépanocytaire et autres hémoglobinopathies, stries angioïdes, vitréorétinopathie exsudative familiale, maladie de Eales, affections traumatiques/chirurgicales de l'œil, ophtalmie sympathique, maladie rétinienne uvéitique, détachement rétinien, trauma, affections causées par le laser, affections causées par une photothérapie dynamique, photocoagulation, hypoperfusion pendant une chirurgie, rétinopathie due au rayonnement, rétinopathie due à un transplant de moelle osseuse, cicatrisation ou inflammation de plaie cornéenne post-chirurgicale, inflammation chirurgicale post-cataracte, troubles prolifératifs de l'œil, rétinopathie vitréenne proliférative et membranes épirétiniennes, rétinopathie diabétique proliférative, troubles infectieux de l'œil, histoplasmose oculaire, toxocarose oculaire, syndrome de l'histoplasmose oculaire présumée (POHS), endophtalmite, toxoplasmose, maladies rétiniennes associées à une infection au VIH, maladie choroïdienne associée à une infection au VIH, maladie uvéitique associée à une infection au VIH, rétinite virale, nécrose rétinienne aiguë, nécrose rétinienne externe progressive, maladies rétiniennes fongiques, syphilis oculaire, tuberculose oculaire, neurorétinite subaigüe unilatérale diffuse, myiase, troubles génétiques affectant l'œil, rétinite pigmentaire, troubles systémiques avec dystrophies rétiniennes associées, cécité crépusculaire stationnaire congénitale, dystrophies des cônes, maladie de Stargardt, fundus flavimaculatus (maladie de Franceschetti), maladie de Best, dystrophie en pattern de l'épithélium pigmentaire rétinien, rétinoschisis lié à l'X, dystrophie du fond d'œil de Sorsby, maculopathie concentrique bénigne, dystrophie cristalline de Bietti, pseudoxanthome élastique, larmes rétiniennes, trous rétiniens, détachement rétinien, trou maculaire, larme rétinienne géante, tumeurs oculaires, maladie rétinienne associée aux tumeurs, hypertrophie congénitale de l'épithélium pigmentaire rétinien, mélanome de l'uvéite postérieure, hémangiome choroïdien, ostéome choroïdien, métastase choroïdienne, hématome combiné de la rétine et de l'épithélium pigmentaire rétinien, rétinoblastome, tumeurs vasoprolifératives du fond oculaire, astrocytome rétinien, tumeurs lymphoïdes intraoculaires, diverses autres maladies affectant la partie postérieure de l'œil, choroïdopathie ponctuée interne, épithéliopathie pigmentaire placoïde multifocale postérieure aiguë, dégénérescence rétinienne myopique, épithélite pigmentaire rétinienne aiguë, inflammation cornéenne post-chirurgicale, blépharite, dysfonctionnement des glandes de méibomius (MGD), cicatrisation de plaie cornéenne, glaucome, occlusion des veines brachiales, dégénérescence maculaire vitelliforme de Best, rétinite pigmenteuse, vitréorétinopathie proliférative (PVR), et tout autre maladie dégénérative soit des photorécepteurs soit de l'épithélium pigmentaire rétinien, une maladie ou affection dermique choisie parmi inflammation dermique, une plaie dermique, cicatrices hypertrophiques, chéloïdes, brûlures, érythème solaire, rosacée, érythème de la peau, dermatite atopique, acné, psoriasis, dermatite séborrhéique, kératoses actiniques, carcinome basocellulaire, carcinome squameux, mélanome, verrues virales, photovieillissement, photolésion, mélasme, hyperpigmentation post-inflammatoire, troubles de la pigmentation, et alopécie, formes à cicatrisation et sans cicatrisation ou la maladie ou affection est choisie parmi une maladie inflammatoire systémique, accident vasculaire cérébral, maladie coronarienne, un trouble cardiovasculaire, angine de poitrine, un syndrome obstructif des voies respiratoires, un trouble neurologique, un trouble du système nerveux central, maladie d'Alzheimer, neuroinflammation, douleur, une infection rétrovirale médiée par VIH, un trouble immunologique, arthrite, polyarthrite rhumatoïde, lupus érythémateux systémique, sclérose en plaques, sepsie, affection abdominale inflammatoire, rectocolite hémorragique, asthme, une maladie allergique et cachexie.
